(19) Europäisches Patentamt
European Patent Office
Office européen des brevets

(11) EP 3 912 014 B1

(12) # EUROPEAN PATENT SPECIFICATION

(45) Date of publication and mention of the grant of the patent:
**03.06.2026 Bulletin 2026/23**

(21) Application number: **19740086.4**

(22) Date of filing: **18.06.2019**

(51) International Patent Classification (IPC):
***G06F 3/01*** *(2006.01)* ***A63F 13/00*** *(2014.01)*
***A61B 5/24*** *(2021.01)* ***A61B 5/00*** *(2006.01)*

(52) Cooperative Patent Classification (CPC):
**G06F 3/015; A61B 5/291; A61B 5/7278**

(86) International application number:
**PCT/JP2019/024855**

(87) International publication number:
**WO 2020/148931 (23.07.2020 Gazette 2020/30)**

(54) **BRAIN-COMPUTER INTERFACE SYSTEM, SYSTEM FOR BRAIN ACTIVITY ANALYSIS, AND METHOD OF ANALYSIS**

HIRN-COMPUTER-SCHNITTSTELLENSYSTEM, SYSTEM ZUR HIRNAKTIVITÄTSANALYSE UND VERFAHREN ZUR ANALYSE

SYSTÈME D'INTERFACE CERVEAU-ORDINATEUR, SYSTÈME D'ANALYSE D'ACTIVITÉ CÉRÉBRALE ET PROCÉDÉ D'ANALYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2019 US 201916250174**

(43) Date of publication of application:
**24.11.2021 Bulletin 2021/47**

(73) Proprietor: **Mitsubishi Electric Corporation**
**Tokyo 100-8310 (JP)**

(72) Inventors:
- **WANG, Pu**
  **Cambridge, Massachusetts 02139 (US)**
- **AKINO, Toshiaki**
  **Cambridge, Massachusetts 02139 (US)**
- **WANG, Ye**
  **Cambridge, Massachusetts 02139 (US)**

(74) Representative: **Pfenning, Meinig & Partner mbB**
**Patent- und Rechtsanwälte**
**An der Frauenkirche 20**
**01067 Dresden (DE)**

(56) References cited:
**WO-A2-2012/153965** **CN-A- 108 433 722**
**US-A1- 2014 171 757** **US-A1- 2014 236 039**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 912 014 B1

## Description

[Technical Field]

The present disclosure relates generally to methods and systems for storing, classifying and analyzing data, and more particularly to correlating brain activity of a user to a predetermined physiological response or a predetermined command.

[Background Art]

Neurophysiological data includes any type of signals obtained from a brain. Such signals may be measured through such tools as EEG (electroencephalogram), which is produced using electroencephalography. Electroencephalography is the neurophysiologic measurement of the electrical activity of the brain (actually voltage differences between different parts of the brain), performed by recording from electrodes placed on the scalp or sometimes in or on brain tissue.

For example, a brain-computer interface (BCI) system can be a system which is meant to permit a user to control an application/device by using only his or her thoughts (more specifically, using neuro-physiological signals detected from the brain or from other nervous tissue). BCI systems are classically based on an electroencephalography (EEG) recorded from sensors attached to the user's head. The EEG is measured and sampled while the user imagines different things (for example, the user moving a body part, such as a leg or hand). Depending on the BCI, particular preprocessing and feature extraction methods are applied to an EEG sample of a certain length, with goal being to reliably detect the limited mind states from the EEG signals or patterns from the EEG samples with a certain level of accuracy.

Conventional BCI systems typically have problems in being reliable, and because of being unreliable have received considerable attention in recent years. Using EEG signals as a non-invasive method has been demonstrated to be a feasible approach in BCI. However, due to a limited understanding of the human brain and the brain's electrical activities, only a limited number of brain states, that are driven by physical or imagined actions, can be reliably detected. For example, some physical or imagined actions can include emotional states to some degree, motor-related EEG changes and wake/sleep modes.

The preprocessed data of conventional BCI's is typically further processed in a feature extraction stage. Wherein these non-invasive BCIs use features derived from individual channels. Some commonly used features include band power (BP), autoregressive (AR) model coefficients, and wavelets. These extracted features are then utilized in the classification stage to allow BCIs to discriminate between different brain states or mental tasks. However, these features do not contain information about causal relationships between channels. US 2014/0171757 A1 discloses a training apparatus using a method of decoding nerve activity and includes a brain activity detecting device and an output device as well as a processing device. A brain-computer interface device, in which a test signal constituted by a brainwave signal is classified into one of a plurality of classes is known from WO 2012/153965 A2. A method of calibrating a direct neural interface system with a determination of a regression function is known from US 2014/0236039 A1. CN 108 433 722 A discloses a portable electroencephalogram collecting device and an application of the portable electroencephalogram collecting device in sports imagery.

Accordingly, there is need to improve BCIs to include features that do contain information about causal relationships between channels, since such information can provide useful features for BCIs.

[Summary of Invention]

The present disclosure relates to correlating brain activity of a user to a predetermined physiological response or a predetermined command of the user to a device. The present invention is set out in the claims. In particular, some methods and systems include measuring directional connectivity by utilizing a new hierarchical VAR model that further exploits nested sparsity patterns across multiple coefficient matrices of the VAR model.

For example, some embodiments of the present invention include methods and systems that perform classification of brain activity based on a connectivity map in time and space. Wherein sparsity is enforced on a determination of the connectivity map in order to more effectively identify the correlations that exist in the multi-dimensional time-series data. For example, by exploiting the sparsity in a vector autoregression (VAR) model having coefficient matrices using hierarchical lag structure of the VAR model. Realized through experimentation is that the hierarchical lag structure can be determined by selecting low-lag coefficients before corresponding high-lag coefficients of the VAR model, thereby shrinking toward low-lag order solutions. Wherein the VAR model can be used for controlling a device with the help of a brain computer interface system (BCI), in that the determined or classified brain activities are classified for controlling the device. Specifically, a classifier can be used to classify the sparse connectivity map as a user's intention or a mental state by the user, to control or impact an operation of the device.

For example, to control or impact the operation of the device, the user's intention or mental state by the user can be communicated from a computer/processor/controller to a control output in an embodiment not covered by the present invention. Wherein the control output can be in communication with an interface of the device that can implement some type of action related to some user's intention or some mental state of the user. The interface of the device may be a physical actuator controller of a muscle movement device in communication with a muscle(s) of the user. Further, the interface of the device could be a computer input device, or the interface could be a device guidance control for a Heating, Ventilation, and Air Conditioning (HVAC) system(s), vehicle or wheel chair, by non-limiting example. Other examples of devices that can be controlled by a control output from a computer/processor/controller can be a spelling application, a neuroprosthesis or a mechanical device related to an interest of the user, i.e. household appliance, device related to the user's daily living habits and routine, etc.

Some realizations of the present disclosure discovered from experimentation support using the utility of data analytics and signal processing for deriving information from brain signals. For example, information derived can represent inner feelings and emotions of a human mind which are usually invisible immeasurable, and unpredictable, which can be predicted with the help of different data analytical models. Brain signals can be detected by using electroencephalogram (EEG) to record the brain

signals and for subsequent processing based on a chosen learning model, such as the VAR model.

The present disclosure utlizes the brain computer interface (BCI) where a mental task in the brain can be determined based on the configuration of the active areas in the brain. BCI systems classifies brain activity and controls a device such as a spelling application, a neuroprosthesis, or a wheelchair. Most non-invasive BCIs rely on the electroencephalogram (EEG) to record brain signals for subsequent classification. For example, the configuration of the active areas in the brain can be determined with the help of available prediction models, i.e. VAR models. Conventional predicton models fail, when the regions in the brain being considered, increase in an amount of brain activity, and the connectivity map becomes too complicated for classification, and thus simply fail. Conventional VAR models also assume a single and universal lag structure that applies across all components, which results in unnecessarily constraining the dynamic relationships across the components. In contrast, the present disclosure focuses on the aforesaid problem, by exploiting sparsity in the VAR coefficients, so as to solve the conventional problems of complexity in the classification of the brain activities, by non-limiting example, in real time, using less computational time and costs, allowing for the methods and systems of the present disclosure to utilized in meeting today's advanced technological demands by users.

The VAR model can determine the brain activity mapping that is an econometric model used to capture linear interdependencies among multiple time series. Also, the VAR model can include a set of variables, where each variable is an equation based on its own lagged values, the lagged values of the other model variables, and an error term. Further still, the VAR model can be useful in determining the connectivity map defining correlations of the active areas in time and spatial domain. Wherein, the present disclosure exploits the sparsity in the VAR coefficient matrices by using a new hierarchical lag structure of the VAR model. The hierarchical lag structure can be determined by selecting low-lag coefficients before corresponding high-lag coefficients of the VAR model, thereby shrinking toward low-lag order solutions.

Some embodiments of the present disclosure perform classification of brain activity based on a connectivity map in time and space, and enforce sparsity on determination of the connectivity map, in order to more effectively identify the correlations that exist in the multi-dimensional time-series data. Such that, the BCI of the present disclosure overcomes conventional BCI system problems, by at least incorporating configurations that contain information about causal relationships between the active areas of the brain, among other ways. Some benefits to the above approach is less computation cost and time, thus making embodiments of the present disclosure advantagous over conventional predicton models that fail.

Causal relationships can refer to a connection of differerent acitive areas when some areas are responsible for the cause and the other areas are the result of the effect. The connectivity serves as a basis to understand the causal relationship, which is at least one goal of connectivity estimation. At least one benefit to better understanding of causal relationships can include, by non-limiting example, identifying or administering new treatments of certain brian diseases and the like, and enhancement of brain perceptions.

For example, some embodiments of the present disclosure measure directional connectivity of brain activity of the user by utilizing the new hierarchical VAR (HVAR) model. The new HVAR model illustrates how to correlate a source activation with the hierarchical VAR model, exploits nested sparsity patterns across multiple coefficient matrices of the VAR model, and uses an inference method to estimate the coefficient matrices with an observed multi-channel EEG signal.

However, achieving such a task of utilizing the new hierarchical was based on many realizations gained through extensive experimentation. At least one realization included enforcing sparsity on the determination of the connectivity map. Firstly, the sparse connectivity map can be more accurate representation of the brain activity. Secondly, the sparse connectivity map can reduce the classification space and can result in smaller (finite) number of various combinations of correlations among active regions of the brain. Wherein, that smaller number of combinations of correlations is easier to classify (with a trained classifier). Also, the aspect of connectivity estimation can be used to assist in better understanding the causal relationships, as noted above, by better understanding causal relationships, many benefits can be taken advantage of, like new treatments of certain brain diseases, enhancement of brain perceptions, etc.

Another realization is that conventional or traditional VAR models assume a single and universal lag structure that applies across all components. While this reduces the computational complexity of model selection, the conventional VAR models administer unnecessarily constrains on the dynamic relationship across the components. Which, causes failure when the regions in the brain being considered, increase in an amount of brain activity, this increase in the amount of brain activity increases the connectivity map, resulting in a connectivity map that becomes too complicated for classification.

Another realization is that sparsity can be enforce by HVAR model that forces low-lag coefficients to be selected before corresponding high-lag coefficients, thereby shrinking toward low-lag order solutions. Wherein, such a shrinking, enforces sparsity on the resulting connectivity map.

Also, learned during experimentation, some experiments that used instantaneous connectivity, was later discovered, to be problematic, that caused *spurious* connectivity between raw EEG channels. Some solutions discovered/realized from experimentation to this problem included: (1) connectivity measures designed to suppress instantaneous effects; (2) inclusion of an instantaneous term in the VAR model; and (3) modeling the EEG as a mixture of sources. In regard to the BCI application, gleaned/realized from experimentation is that connectivity measures need to be extracted online from the on-going EEG. However, in practice/experimentation, we discovered that many connectivity measures are extracted from VAR models, which are fitted to data that contains multiple realizations of the EEG time series (multiple trials). Connectivity estimation with less trails (or a single trail to the extreme) was found to be inherently more difficult, because only a fraction of the data is available for model fitting. Thus, these approaches resulted in less experimentation and not a primarily focus of the present disclosure. However, under certain particular circumstances, along with special aspects, the methods of the present disclosure may be considered and utilized with the above approaches, so as to achieve an approach level of estimation accuracy of brain connectivity maps, that can be similar to the methods and systems of the present disclosure. For instance, to estimate a connectivity map for brain reaction to a transient behavior, e.g., a shock, corresponding observable EEG signals are short and, hence, utitlizing more structural model is necessary to highlight prominent source connections while ignoring marginally insignificant souce connections.

Contemplated is that the present disclosure can be used in technologies including, by non-limiting example: Intelligent brain-machine interface (BMI) technologies; Sensor-empowered HVAC system technologies; Accident prevention for intelligent transport system (ITS) technologies; factory automation (FA) technologies; Smart home for health monitoring system technologies; energy management technologies; and security related technologies.

The invention is defined by the independent claims. Preferred embodiments are disclosed in the dependent claims.

The presently disclosed embodiments will be further explained with reference to the attached drawings. The drawings shown are not necessarily to scale, with emphasis instead generally being placed upon illustrating the principles of the presently disclosed embodiments.

[Brief Description of Drawings]

[FIG. 1A]
FIG. 1A is a flow diagram illustrating some method steps for implementing a method, according to some embodiments of the present disclosure.

[FIG. 1B]
FIG. 1B is a block diagram illustrating some components that can be used for implementing the systems and methods, according to some embodiments of the present disclosure.

[FIG. 1C]
FIG. 1C is a schematic diagram illustrating multiple active voxels (areas) of a brain of a user where scalp potential signals are obtained, according to some embodiments of the present disclosure.

[FIG. 1D]
FIG. 1D is a schematic diagram illustrating a Vector Autoregressive (VAR) Modeling of Direct Source Signals (scalp potential signals), such that the VAR model assumes that a multi-channel scalp potential signal s(t) is a linear combination of previous p samples of multi-channel scalp potential signals s(t-$\tau$) plus independent noise n(t), according to some embodiments of the present disclosure.

[FIG. 1E]
FIG. 1E is a schematic diagram illustrating Sparsity in VAR Coefficient Matrix $B_s(\tau)$, according to some embodiments of the present disclosure.

[FIG. 2A]
FIG. 2A is a schematic diagram illustrating a Signal Generative Model that starts with FIG. 1E regarding the connectivity map when $T$ = **1,** then to FIG. 1C & FIG. 1D regarding the unobservable scalp potential signals, and further to, and additionally, observable brain signals (EEG), according to some embodiments of the present disclosure.

[FIG. 2B]

FIG. 2B is a schematic diagram illustrating EEG channels of the observable signals (EEG) of FIG. 2A, according to some embodiments of the present disclosure.

[FIG. 2C]

FIG. 2C is a schematic diagram illustrating connectivity estimation steps, beginning with the observable brain signals (EEG) where a Principle Component Analysis (PCA) or Blind Source Separation (BSS) can be utilized, then to the unobservable scalp potential signals, where a connectivity estimation can begin to identify the connectivity map, according to some embodiments of the present disclosure.

[FIG. 2D]

FIG. 2D is a block diagram illustrating steps of a method, that includes step 1 the observable brain signals (EEG) from sensor locations attached to the head of the user, step 2 mapping from the EEG to Scalp Potentials (PCA) and step 3 the hierarchical sparse connectivity map estimation a key aspect of the present disclosure, of the schematic diagram of FIG. 2B, and in addition step 4 the classification of mental command, along with step 5 controlling a device, according to embodiments of the present disclosure.

[FIG. 3A]

FIG. 3A is a schematic diagram of conventional connectivity estimation using a VAR Model without any sparsity constraints.

[FIG. 3B]

FIG. 3B is a schematic diagram of the conventional connectivity estimation using the VAR Model without any sparsity constraints, that illustrates there are no constraints on the VAR coefficient matrices.

[FIG. 3C]

FIG. 3C is a schematic diagram of a hierarchical VAR model that imposes hierarchical sparsity constrains on VAR coefficient matrices, according to embodiments of the present disclosure.

[FIG. 3D]

FIG. 3D is a schematic diagram of the hierarchical VAR model that imposes hierarchical sparsity constrains on VAR coefficient matrices, illustrating zero patterns over all delay lags having hierarchical structures, such that the second row starts to be zeros when the delay lag $\tau > 2$, according to embodiments of the present disclosure.

[FIG. 3E]

FIG. 3E is a schematic diagram of the conventional VAR model illustrating a universal lag structure of the VAR model with four channels and four lags.

[FIG. 4]

FIG. 4 is a schematic illustrating some aspects of a system that includes selective actuation of a control action in different control applications of the system, according to embodiments of the present disclosure.

[FIG. 5A]

FIG. 5A is a schematic illustrating a computing apparatus, processor that can be used to implement some techniques of the methods, systems and devices, according to embodiments of the present disclosure.

[FIG. 5B]

FIG. 5B is a schematic illustrating a computing apparatus configured for traveling, that can include one or more processor that can be used to implement some techniques of the methods, systems and devices, according to embodiments of the present disclosure.

While the above-identified drawings set forth presently disclosed embodiments, other embodiments are also contemplated, as noted in the discussion. This disclosure presents illustrative embodiments by way of representation and not limitation. Numerous other modifications and embodiments can be devised by those skilled in the art which fall within the scope of the principles of the presently disclosed embodiments.

[Description of Embodiments]

FIG. 1A is a flow diagram illustrating some method steps for implementing a method, according to embodiments of the present disclosure. For example, a method analysis 100A can include steps, such as step 111 that includes operating an array of electroencephalography (EEG) electrodes and/or magnetoencephalography (MEG) electrodes for receiving EEG signals and/or MEG signals from a brain of a user.

Step 113 of method 100A of FIG. 1A can include using an encoder for encoding the received the EEG signals and/or the MEG signals to produce a sparse connectivity map of correlations among active regions of the brain, wherein the sparse connectivity map specifies more zero correlations than non-zero correlations between various active regions of the brain.

Step 115 of FIG. 1A can include using a classifier to classify the sparse connectivity map as an intended predetermined physiological response intended by the user.

Step 117 of FIG. 1A can include using a controller to communicate a control signal for actuating the control action according to the intended predetermined physiological response to a device.

Some embodiments of the present disclosure provide unique aspects over conventional brain-computer interface (BCI) systems, by non-limiting example, that include: (1) being more effective in identifying correlations that exist in the multi-dimensional time-series data, when compared to conventional prediction models/systems when the regions in the brain being considered, increase in an amount of brain activity, and the connectivity map becomes too complicated for classification, and thus the conventional prediction models/systems simply fail; (2) exploiting sparsity in the VAR coefficients, so as to solve the conventional problems of complexity in the classification of the brain activities, by overcoming conventional prediction methods that assume a single and universal lag structure that applies across all components, which results in unnecessarily constraining the dynamic relationships across the components. In particular, the exploiting of the sparsity in the VAR coefficient matrices can be by using a new hierarchical lag structure of the VAR model, such that the hierarchical lag structure can be determined by selecting low-lag coefficients before corresponding high-lag coefficients of the VAR model, thereby shrinking toward low-lag order solutions, whereas the conventional prediction models; and (4) the BCI of the present disclosure overcomes conventional BCI system problems, by at least incorporating configurations that contain information about causal relationships between the active areas of the brain, such that some benefits can be less computation cost and time.

Other benefits, among many, is that the present disclosure, by imposing more structural constrains, can enhance significant connectivity links and suppress insignificant links. Therefore, the causal relationship among acitive areas will be easier to infer and better understood. It also requires less EEG signals (less acquisition time and less EEG sensors) to achieve the same level of estimation performance.

Some embodiments can include several aspects such as including: the translation of signals into a representation that allows for their manipulation and comparison; comparison of classes of signals to ascertain and extract characteristic signatures; creation of a detector/classifier to recognize signatures in a way that is robust in view of noise and environmental factors; and localization of detected signatures, if necessary. Such that, some embodiments include aspects that provide for processing, analyzing, and comparing, of brain signals in order to facilitate signature detection. The process can begin with collecting brain data that is representative of the signals to be detected. The data can be normalized so that individual recordings are approximately comparable, and divided into classes. Each class can preferably comprise multiple recordings of a particular event or state of interest.

FIG. 1B is a block diagram illustrating some components that can be used for implementing the systems and methods, according to embodiments of the present disclosure. For example, method 100B can include the hardware processor(s) or encoder(s) 11 in communication with a sensor 2 or sensors, such as an EEG sensor, that collects data including brain activity 3 from a user 1. The hardware processor 11 is in communication with a computer storage memory, i.e. memory 10, such that the memory includes stored data, including algorithms (such as classification and connectivity estimation), instructions, historical data (pre-saved connectivity patterns) and other data (other personal data such as health records related to brain), that can be implemented by the hardware processor 11. The memory 10 can be connected to the input interface 9, the brain activity 3. An output interface 14 can be connected to the processor or encoder 11 and data 8.

Optionally, the hardware processor 11 can be connected to the input interface 9, a user interface 12 and external memory 17. Also optionally, the hardware processor 11 can be connected to a network-enabled server 13 connected to a client device 15.

It is contemplated the hardware processor 11 can include two or more hardware processors depending upon the requirements of the specific application, wherein the processors can be either internal or external. Certainly, other components may be incorporated with method 100B including output interfaces and transceivers, among other devices.

Contemplated is that the hardware processor 11 can receive or send data to a network(s) 7, in communication with a data source(s) 4, a storage device(s) 6 and a computer(s) or a laptop(s) 5, via the output interface 14.

Further, the hardware processor 11 can be connected to a controller 19 that is connected to a device 21. Wherein the device 21 can be in communication with a HVAC system 31, a wheelchair 32 or an electronic device 33.

Also, the device 21 can be related to technologies including: Intelligent brain-machine interface (BMI) technologies; Sensor-empowered HVAC system technologies; Accident prevention for intelligent transport system (ITS) technologies; factory automation (FA) technologies; Smart home for health monitoring system technologies; energy management technologies; and security related technologies.

FIG. 1C is a schematic diagram illustrating multiple active voxels (areas) of a brain of a user where scalp potential signals are obtained, according to embodiments of the present disclosure. For example, each active voxel (area) can be in communication with a sensor/electrode that provides scalp potential signals, wherein an active voxel 41A provides scalp potential signals 41B, active voxel 43A provides scalp potential signals 43B, active voxel 45A provides scalp potential signals 45B, and active voxel 47A provides scalp potential signals 47B.

These scalp potential signals 41B, 43B, 45B and 47B vs time are normally unobservable and considered as direct source signals from which to understand the connectivity for a given brain function.

FIG. 1D is a schematic diagram illustrating a Vector Autoregressive (VAR) Modeling of Direct Source Signals (scalp potential signals), such that the VAR model assumes that a multi-channel scalp potential signal s(t) is a linear combination of previous *p* samples of multi-channel scalp potential signals s(t-τ) plus independent noise n(t), according to embodiments of the present disclosure. For example, the formula below includes

$$\mathbf{s}(t) = \mathbf{B}_s(1)\mathbf{s}(t-1) + \mathbf{B}_s(2)\mathbf{s}(t-2) + \cdots + \mathbf{B}_s(p)\mathbf{s}(t-p) + \mathbf{n}(t) \quad \text{(Eq.1)}$$

Where character reference "$B_s(\tau)$" represents VAR coefficient matrices for a given lag τ,"$B_s(1)s(t\text{-}1)$" represents the signal component of s(t) contributed from s(t-1),
"$B_s(2)s(t\text{-}2)$" represents the signal component of s(t) contributed from s(t-2),
"$B_s(p)s(t\text{-}p)$" represents the signal component of s(t) contributed from s(t-p).

Also, voxel number 1 (41A of FIG. 1D) correlates to scalp potential signal 41B, $S_1(t)$, voxel number 2 (43A of FIG. 1D) correlates to scalp potential signal 43B, $S_2(t)$, voxel number 3 (43A of FIG. 1D) correlates to scalp potential signal 43B, $S_3(t)$, and voxel number 4 (47A of FIG. 1D) correlates to scalp potential signal 47B, $S_4(t)$.

FIG. 1E is a schematic diagram illustrating sparsity in VAR Coefficient Matrix $B_s(\tau)$, according to embodiments of the present disclosure. FIG. 1E also shows the connectivity map when t = 1, wherein the circles denote the voxel number 1 (41A of FIG. 1E), voxel number 2 (43A of FIG. 1E), voxel number 3 (45A of FIG. 1E), and voxel number 4 (47A of FIG. 1E). Further, the edges of the circles 1-4 denote connectivity or causality.

For example, character reference number 51 represents no connectivity from voxel 2 (43A of FIG. 1E) to voxel 1 (41A of FIG. 1E), character reference number 53 represents connectivity from voxel 4 (47A of FIG. 1E) to voxel 1 (41A of FIG. 1E), and character reference number 55 represents connectivity from voxel 4 (47A of FIG. 1E) to voxel 3 (45A of FIG. 1E). Wherein the box like diagram of FIG. 1E illustrates the corresponding zero patterns in the VAR coefficient matrix $B_s(1)$. In this case, $B_s(1)$ is a 4 x 4 symmetric matrix.

FIG. 2A is a schematic diagram illustrating a Signal Generative Model that incorporates the connectivity map of FIG. 1E and unobservable scalp potential signals of FIG. 1C and FIG. 1D, as well as in addition observable brain signals (EEG), according to embodiments of the present disclosure. For example, the Signal Generative Model includes: (1) the connectivy map 211, (2) the scalp potential signals 213 from voxel 1 (241A) corresponds to signal 241B, voxel 2 (243A) corresponds to signal 243B, voxel 3 (245A) corresponds to signal 245B, and voxel 4 (247A) corresponds to signal 247B, these are not observable scalp potential signals; and (3) the observable brain signals (EEG) 215.

Further, the multiple EEG sensors/electrodes placed on the scalp collect the observable brain signals (EEG) 215. At least one purpose to use the observable brain signals (EEG) 215 is to estimate the connectivity among active brain areas for a given action or a sequence of brain events.

FIG. 2A is a schematic diagram further illustrates the signal generative model. Equivalently, for a brain activity and a corresponding connectivity map, it generates scalp potential signals 213 at a certain number of voxels 241A, 243A, 245A, 247A. These scalp potential signals are usually not directly observable by sensors. Instead, these scalp potential signals are mapped into observale EEG signals. With this signal generative model, our goal is to reverse this flowchart to estimate the connectivity map (at the left side) with (maybe limited) observable EEG signals.

FIG. 2B is a schematic diagram further illustrates EEG channels or sensor/electrode locations attached to a head of the user of FIG. 2A, according to embodiments of the present disclosure. In particular, a sensor/electrode capturing brainwave activity can be called an EEG channel, such that some EEG systems can have as few as a single channel or as many as 256 channels. Electrode placement on the head adheres to a formal standard called the 10/20 system or International 10/20 system. Further, the number of the EEG channels or sensor locations may be less than a number of active voxels 213 of FIG. 2A. Further still, the layout of EEG electrodes can include 45 electrodes/sensors, such that the locations of the EEG electrodes correspond to the extended international 10-20 system.

FIG. 2C is a schematic diagram illustrating connectivity estimation steps, beginning with the observable brain signals (EEG) 215 where a Principle Component Analysis (PCA) or Blind Source Separation (BSS) can be utilized, then to the unobservable scalp potential signals 213, where a connectivity estimation can begin to identify the connectivity map 211, according to some embodiments of the present disclosure. Noted, the scalp potential signals 213 from voxel 1 (241A) corresponds to signal 241B, voxel 2 (243A) corresponds to signal 243B, voxel 3 (245A) corresponds to signal 245B, and voxel 4 (247A) corresponds to signal 247B, these are not observable scalp potential signals.

FIG. 2D is a block diagram illustrating steps of a method, that includes step 1 the observable brain signals (EEG) from sensor locations attached to the head of the user, step 2 mapping from the EEG to Scalp Potentials (PCA) and step 3 the hierarchical sparse connectivity map estimation a key aspect of the present disclosure, of the schematic diagram of FIG. 2B, and in addition step 4 the classification of mental command, along with step 5 controlling a device, according to embodiments of the present disclosure.

For example, step 1 or 201 includes obtaining brain signals (EEG) from sensors or electrodes attached to the user's head.

Step 2 or 203 is the mapping from EEG to scalp potentials (PCA or BSS). This can be done by performing the singular value decomposition and keeping the principle singluar vectors. On the other hand, BSS performs the signal decomposition by assuming the scalp potential signals at different voxels are independent.

Step 3 or 205 includes the Hierarchical Sparse Connectivity Map Estimation. Noted, is that steps 211, 214 and 215 of FIG. 2C, are converted to a block diagram of FIG. 2D, that correlate to steps 201, 203 and 205, respectively.

Referring to step 205 of FIG. 2D, the methods and systems of the presnt disclosure measure directional connectivity utilizing a new hierarchical VAR model (HVAR) that further exploits nested sparsity patterns across the multiple coefficient matrices of the VAR model. The new HVAR model illustrates how to correlate a source activation with the hierarchical VAR model, exploits nested sparsity patterns across multiple coefficient matrices of the VAR model, and uses an inference method to estimate the coefficient matrices with an observed multi-channel EEG signal. See FIG. 3E regarding a further explanation of the HVAR model.

Still referring to step 205 of FIG. 2D, the methods and systems enforce sparsity on the determination of the connectivity map. Firstly, the sparse connectivity map can be more accurate representation of the brain activity. Secondly, the sparse connectivity map can reduce the classification space and can result in smaller (finite) number of various combinations of correlations among active regions of the brain. Wherein, that smaller number of combinations of correlations is easier to classify (with a trained classifier). Also, the aspect of connectivity estimation can be used to assist in better understanding the causal relationships, as noted above, by better understanding causal relationships, many benefits can be taken advantage of, like new treatments of certain brain diseases, enhancement of brain perceptions, etc. At least one realization of some methods and systems is that sparsity can be enforce by HVAR model that forces low-lag coefficients to be selected before corresponding high-lag coefficients, thereby shrinking toward low-lag order solutions. Wherein, such a shrinking, enforces sparsity on the resulting connectivity map.

Another realization is that conventional or traditional VAR models assume a single and universal lag structure that applies across all components. While this reduces the computational complexity of model selection, the conventional VAR models administer unnecessarily constrains on the dynamic relationship across the components. Which, causes failure when the regions in the brain being considered, increase in an amount of brain activity, this increase in the amount of brina activity increase the connectivity map, resulting in a connectivity map that becomes too complicated for classification. See FIG. 3A for a further explanation of the conventional VAR model.

Optionally, step 207 can include the classification of the mental command according to known processing approaches.

Also optionally, step 209 can include controlling a device, based in part, using the classified mental command of step 207. See FIG. 4 regarding a further explanation of controlling a device.

Contemplated is other steps that may be implemented after step 205 other than steps 207 and 209 For example, the connectivity map (i.e. the measure of directional connectivity utilizing the new hierarchical VAR model (HVAR)) can be used for other purposes, other than for device control. For example, the connectivity map can simply be used for diagnosis of certain brain malfunction, predict human intention(s), estimate the mental stress, etc.

FIG. 3A is a schematic diagram of conventional connectivity estimation using a VAR Model without any sparsity constraints. It first uses the PCA to map the observable EEG signals into a compressed space due to limited number of samples. Then it applies an estimation algorithm based on the conventional VAR model without any structural assumptions on VAR coefficient matrices $B_s(\tau)$.

FIG. 3B is a schematic diagram of the conventional connectivity estimation using the VAR Model without any sparsity constraints, that illustrates there are no constraints on the VAR coefficient matrices.

**(Vector Autoregressive Modeling of Connectivity)**

Learned through experimentation, is that the time-varying neural current density responsible for the EEG scalp potentials can be appropriately modeled by a discrete set of *K* signal generators. Denote s ($t$) = $[s_1(t),\cdots,s_K(t)]^T$ the multivariate activation pattern of those generators at time instant t. Also assume that those source activation patterns are well described by the VAR model of order *p*, at least for a certain temporal range $t = 1,2,\cdots,L$.

$$\mathrm{s}(t) = \sum_{\tau=1}^{p} \mathbf{B}_s(\tau)\mathrm{s}(t-\tau) + \mathrm{n}(t) \qquad \text{(Eq. 1)}$$

Where $B_s(\tau)$, $\tau = 1, \cdots, p$, are the coefficient matrices of the VAR model and $n(t) = [n_1(t),\cdots,n_K(t)]^T$ represents the corresponding multivariate residual (innovation) process. We assume that each EEG generator is a source of independent activity in the sense that the elements of the residual vector n(*t*) behave like mutually independent non-Gaussian random variables. Causal relationships between EEG sources are therefore exclusively produced by time-lagged axonal propagation of macroscopic neural behavior among distant regions of the brain modeled by the coefficient matrices $B_s(\tau)$, $\tau = 1, \cdots, p$.

Furthermore, the multichannel EEG signal *x*(*t*) recorded at time instant t using M electrodes is a multivariate signal s(t) satisfying:

$$\mathbf{x}(t) = \mathbf{\Phi}\mathbf{s}(t) + \boldsymbol{\eta}(t) + \mathbf{\Phi}_{\text{noise}}\mathbf{e}(t) \qquad \text{(Eq. 2)}$$

where $\Phi$ is an unknown $M \times K$ leadfield matrix modeling the volume conduction from the location of the sources to the scalp electrodes, $\eta(t) = [\eta_1(t),\cdots,\eta_M(t)]^T$ denotes the white Gaussian measurement noise at electrodes, and the term $\Phi_{noise}e(t)$ represents the contribution of noisy EEG sources (biological noise). For high-density EEG recordings, we assume $M \geq K$, i.e., the number of electrodes is larger than the number of neural sources contributing to the scalp EEG. By combining Eq. (1) and the noiseless version of Eq. (2), we obtain that the observed EEG follows the VAR model:

$$\begin{aligned}\mathbf{x}(t) &= \sum_{\tau=1}^{p} \mathbf{\Phi}\mathbf{B}_s(\tau)\mathbf{\Phi}^{+}\mathbf{x}(t-\tau) + \mathbf{\Phi}\mathbf{n}(t) \\ &= \sum_{\tau=1}^{p} \mathbf{B}_x(\tau)\mathbf{x}(t-\tau) + \mathbf{v}(t)\end{aligned} \qquad \text{(Eq. 3)}$$

where $[\cdot]^+$ denotes the Moore-Penrose pseudo-inversion.

Granger causality studies applied to human EEG signals have typically used estimates of $B_x(\tau)$ to measure directional flows of macroscopic synaptic activity between scalp EEG electrodes, with the implicit assumption that causal relationships between electrodes imply functional connectivity between their respective underlying cortical regions. This is equivalent to assuming that $B_x(\tau) \approx B_s(\tau)$. Indeed, this assumption is not valid in general because the VAR model that best fits the observed EEG data might be strongly affected by volume conduction effects (the matrix $\Phi$ in Eq. (3)).

**(Statistical Measures for Connectivity)**

Regarding the statistical measures for connectivity, the connectivity measures can be defined based on the VAR model of the source activation in Eq. (1). Specifically, the spatio-temporal spectral properties of the EEG sources can be uncovered by transforming (1) to the frequency domain:

$$\mathbf{S}(f) = \left(\mathbf{I} - \sum_{\tau=1}^{p} \mathbf{B}_s(\tau)e^{-j2\pi\tau f/fs}\right)^{-1} \mathbf{N}(f) \triangleq \mathbf{H}(f)\mathbf{N}(f) \qquad \text{(Eq. 4)}$$

where N(*f*) is the Fourier transform of the residual process n(*t*), $f_s$ is the sampling frequency, and H(*f*) is the transfer function matrix H(*f*) describing transfer of spectral properties (coherent links) between EEG sources.

One measure for the directed connectivity is the DTF:

$$\mathbf{DTF}_{ij}(f) = \frac{|\mathbf{H}_{ij}(f)|^2}{\mathbf{H}_{i,:}(f)\mathbf{H}_{i,:}^H(f)} \quad \text{(Eq. 5)}$$

while the full-frequency DTF (ffDTF) is defined as

$$\mathbf{ffDTF}_{ij}(f) = \frac{|\mathbf{H}_{ij}(f)|^2}{\sum_f \mathbf{H}_{i,:}(f)\mathbf{H}_{i,:}^H(f)} \quad \text{(Eq. 6)}$$

**(HVAR MODEL: Hierarchical Lag Structures)**

The cross-spectral density is obtained by

$$\mathbf{S}(f) = \mathbf{H}(f)\mathbf{\Sigma}_n\mathbf{H}^H(f) \quad \text{(Eq. 7)}$$

where $\Sigma_n$ is the covariance matrix of n(*z*). The COH is the normalized cross-spectral density

$$\mathbf{COH}_{ij}(f) = \frac{|\mathbf{S}_{ij}(f)|}{\sqrt{\mathbf{S}_{ii}(f)\mathbf{S}_{jj}(f)}} \quad \text{(Eq. 8)}$$

Other variants of the COH are the imaginary coherence (iCOH) and the partial coherence (pCOH):

$$\mathbf{iCOH}_{ij}(f) = \frac{\Im\{\mathbf{S}_{ij}(f)\}}{\sqrt{\mathbf{S}_{ii}(f)\mathbf{S}_{jj}(f)}} \quad \text{(Eq. 9)}$$

$$\mathbf{pCOH}_{ij}(f) = \frac{|\mathbf{G}_{ij}(f)|}{\sqrt{\mathbf{G}_{ii}(f)\mathbf{G}_{jj}(f)}} \quad \text{(Eq. 10)}$$

where $\mathbf{G}_{ij}(f) = \mathbf{A}^H(f)\mathbf{\Sigma}_n^{-1}\mathbf{A}(f)$ . with A(*f*) = $H^{-1}$(*f*).

In a short summary, to estimate the above connectivity measures, one has to estimate the transfer matrix H of Eq. (4), which is a function of the coefficient matrices of the VAR model of Eq. (1). Therefore, the problem of interest is to estimate $B_s(\tau), \tau = 1,\cdots,p$, from the observed EEG signal x(*t*) in Eq. (2).

FIG. 3C is a schematic diagram of a hierarchical VAR model that imposes hierarchical sparsity constrains on VAR coefficient matrices, according to embodiments of the present disclosure. In particular, the methods and systems of the present disclosure provide for measuring directional connectivity by utilizing a new hierarchical VAR model (HVAR) 355 which further exploits nested sparsity patterns across the multiple coefficient matrices of the VAR model. The methods and systems of the present disclosure also correlate the source activation with the HVAR model 355 with capability of exploiting the nested sparsity across the coefficient matrices, and an inference method to estimate the coefficient matrices with the observed multi-channel EEG signal.

FIG. 3D is a schematic diagram of the hierarchical VAR model that imposes hierarchical sparsity constrains on VAR coefficient matrices, illustrating zero patterns over all delay lags having hierarchical structures, such that the second row starts to be zeros when the delay lag $\tau > 2$, according to embodiments of the present disclosure. To be precise, the nested sparsity patterns refer to that the zero patterns in $B_s(\tau_1)$ enforce that $B_s(\tau > \tau_1)$ are also zeros at the same location.

Besides, $B_s(\tau > \tau_1)$ can be zeros at other locations where $B_s(\tau_1)$ have non-zero connectivities. For example, the second row of $B_s(\tau_1=2)$ is zero which automatically implies that $B_s(\tau > \tau_1)$ are also zeros at the second row, while the third row of $B_s(\tau > \tau_1)$ is zero where $B_s(\tau_1=2)$ is non-zero.

FIG. 3E is a schematic diagram of the conventional VAR model illustrating a universal lag structure of the VAR model with four channels and four lags. In this case, the zero patterns is not a function of $\tau$. That is, a zero in any $B_s(\tau)$ implies zero patterns at all other $B_s(\tau)$. For example, the second row of $B_s(\tau=2)$ is zero. This enforces all second rows for $B_s(\tau<2)$ and $B_s(\tau>2)$ to be zeros.

The conventional VAR model assumes Eq. (1) without structural constrains on $B_s$. However, the conventional VAR model assumes a *universal* lag structure that applies across all components (see FIG. 2B). While the conventional VAR model reduces the computational complexity of model selection, the conventional VAR model unnecessarily constrains the dynamic relationship across the components, e.g., signals at voxels $s_1(t)$, $s_2(t)$. On the other hand, the HVAR model of the present disclosure forces low-lag coefficients to be selected before corresponding high-lag coefficients, thereby shrinking toward low-lag order solutions. This can lead to better connecitivity estimation performance under the scanerio of limited EEG signals and highlight significant connectivities while suppressing insignificant connectivities into zeros.

Specifically, we define the parameter $B_{ij}(l)$ controls the dynamic dependence of the *i*-th component of $s(t)$ on the *j*-th component of $s(t-l)$ , where $l = 1,\cdots,p$. Then the $K \times K$ element-wise coefficient lag matrix L is defined by

$$\mathbf{L}_{ij} = \max\{l, \mathbf{B}_{ij}(l) \neq 0\} \qquad \text{(Eq. 11)}$$

Therefore, each $L_{ij}$ denotes the maximal coefficient lag (maxlag) for component *j* in the regression model for component *i*. In particular, $L_{ij}$ is the smallest *l* such that $Lij(l+1:p) = 0$. In general, the maxlag matrix L is not symmetric. In the conventional VAR model of FIG. 3E, we have

$$= \begin{bmatrix} 3 & 3 & 3 & 3 \\ 1 & 1 & 1 & 1 \\ 2 & 2 & 2 & 2 \end{bmatrix}, \text{ for the HVAR lag structure in FIG. 3D} \qquad \text{(Eq. 12)}$$

where $L_{ij} = L$ for all *i* and *j*, meaning that there is a universal maxlag that is shared by every pair of components. More flexible hierarchical lag structures can be described by the maxlag matrix L. For instance, in FIG. 3D

$$= \begin{bmatrix} 3 & 3 & 3 & 3 \\ 1 & 1 & 1 & 1 \\ 2 & 2 & 2 & 2 \end{bmatrix}, \text{ for the HVAR lag structure in FIG. 3D} \qquad \text{(Eq. 13)}$$

which is a special *component-wise* lag structure, where $L_{ij} = L_i$. More generally, we can a *element-wise* lag structure, or, equivalently, a completely flexible hierarchical lag structure in which the elements of L are not associated with each other.

**(Hierarchical Group LASSO)**

Given the above hierarchical lag structure of the VAR model, the problem of interest is to estimate the VAR coefficient matrices $B_s(l)$ and recover the residual signal. In order to exploit the nested sparsity in the VAR coefficient matrices, we can use the group LASSO with a nested group structure. Particularly, the group lasso is a sum of Euclidean norms and is used to encourage groups of parameters to be set to zero simultaneously. Using nested groups leads to hierarchical sparsity constraints in which one set of parameters being zero implies that another set is also zero. This penalty has been applied to multiple statistical problems including regression models with interactions, covariance estimation, additive modeling, and time series.

Mathematically, we have the following group LASSO for both component-wise and element-wise lag structures. For the component-wise lag structure, the group LASSO is given as

$$\min_{\mathbf{B}(\tau)} \sum_{t=1}^{T} \|\mathbf{s}(t) - \sum_{\tau=1}^{p} \mathbf{B}(\tau)\mathbf{s}(t-\tau)\|_2^2 + \lambda \sum_{i=1}^{K} \sum_{l=1}^{p} \|\mathbf{B}_i(l:p)\|_2 \quad \text{(Eq. 14)}$$

where $B_i(l{:}p)$ denotes a concatenated vector of dimension $\mathbb{R}^{1\times K(p-l+1)}$ which stacks the $i$-th row of the ($p$-$l$+1) VAR coefficient matrices $B(\tau)$, $\tau = l,l, + 1,\cdots,p$ together. On the other hand, for the element-wise lag structure, the group LASSO is given as

$$\min_{\mathbf{B}(\tau)} \sum_{t=1}^{T} \|\mathbf{s}(t) - \sum_{\tau=1}^{p} \mathbf{B}(\tau)\mathbf{s}(t-\tau)\|_2^2 + \lambda \sum_{i=1}^{K} \sum_{j=1}^{K} \sum_{l=1}^{p} \|\mathbf{B}_{ij}(l:p)\|_2 \quad \text{(Eq. 15)}$$

Given the above formulations of the group LASSO, one can find different solvers. such as the proximal gradient method, an extension of traditional gradient descent methods to non-smooth objective functions. An accelerated version of the proximal gradient method, referred to as the Fast Iterative Soft-Thresholding Algorithm (FISTA), can be used for the above group LASSO methods.

FIG. 4 is a schematic illustrating some aspects of a system that includes selective actuation of a control action in different control applications of the system, according to embodiments of the present disclosure. For example, some applications of the system can include different types of responsive steps to an operating system regarding monitoring and control actions.

For example, the classification of the mental command of step 207 of FIG. 2C obtained from the hierarchical sparse connectivity map estimation of step 205 of FIG. 2C, begins with the observable brain signals, e.g., EEG, 215 of FIG. 2B, to estimate the connectivity among active brain areas for a given action or a sequence of brain events. In other words, the processing begins with the user 1 of FIG. 1B, and sensors or transducers 2 of FIG. 1B and leads eventually through the system to a control output(s) 401, 403, 405, 407, 409 of FIG. 4. As an example, the brain activity 3 of FIG. 1B can be sent to the hardware processor/encoder 11 of FIG. 1B, which is processed by the processor 11, and sent to a controller 19 of FIG. 1B, such that the controller sends a control output 401, 403, 405, 407, 409 of FIG. 4, to monitoring 417 and control actions of the device 421, 423, 425, 427, 429, 433, 435 of FIG. 4. Generally, an actuation interface 411, 413, 415, 417, 419 can be configured, depending on the intended application, can be connected to the control output 401, 403, 405, 407, 409 to address appropriate administration of the control action. The control action can be selected according to the selected response(s) for which the classification of the mental command was derived via the hierarchical sparce connectivity map estimation 205 of FIG. 2C, for example, the selected response can be a physical related response or some other type of response.

Still referring to FIG. 4, at least one control output 401 activates a physical actuator controller 411, that can remotely control prosthetic limbs 421, remotely control robotic devices (not shown), or some other device related to the user's movability, health or daily habits. In particular, the system can include initiating the user to move a limb, or asking the user to imagine moving a limb, wherein the limb may be replaced with a mechanical device, i.e. robotic device, or some other device related to the user's movability, health or daily habits. Also possible is that the initiating by the system to the user can be for the user to make a sound, a verbal phrase or perform some other response/action to associated with the initiated control of the specific device. For example, the system can be trained based on the system initiating to the user to do something, such that after training and further classification of the mental commands, the processing system 11 of FIG. 1B can operate to detect when similar signals arise in the user's brain and classify them to perform the specific physical actuator motion, and the like.

Another control output 403 can be an input to a computer via a computer input device 413, wherein training might include asking the user to do or imagine doing some initiated requests such as thinking of specific words or specific phrases/letters, etc. Also possible is that some initiated requests can include the user talking, moving a body part, which is related to the specific control action of the specific device. At least one goal of some of the methods and systems of the present disclosure, is that after training and the classification of the mental command (i.e. derived from the hierarchical sparse connectivity map estimation 205 of FIG. 2C), the processing system 11 of FIG. 1B can operate to detect when similar signals arise in the user's brain, classify them, and generate a specific input signal. Such that the above methods and system can allow the user to communicate with and control the inputs of the computer without physical contact or manipulation.

Another control output 405 can be a control signal for a device guidance control 415 for a HVAC system 425, a vehicle 427 or a wheel chair 429. Some advantages of utilizing the methods and systems of the present disclosure is that user's may be able to control the environmental conditions within an area they are located via the HVAC system 425. Other advantages may be users can control their vehicle 427, or users that are handicapped can control their wheelchairs 429 or other transportation devices. For example, other transportation devices can include modes of transportation in air, land or water, that move the user via guidance systems that are controlled by the user when the user is located in the specific mode of transportation. Contemplated is that the normal devices to control the different modes of transportation may be replaced or supplemented with, depending upon the application, an interface directed by the user's brain signals from the user based upon the methods and systems of the present disclosure.

Still another control output 407 can be a brain state monitoring interface 417, that can be utilized as an indicator signal which reflects brain states of interest of the user to medical personal (or other personnel interested in the safety of the user). Wherein a measured state of consciousness, pain or a specific medical event, such as head trauma, concussion, coma, and seizure, can be alerted to the medical personnel. Further, the methods and systems can be utilized to monitor the signals of the user's brain, classify a mental command, according to a degree of a measured state of the user (i.e. consciousness, pain or a specific medical event, etc.), wherein the classification of the mental command can be utilized to assist medical personnel monitoring of the user's level of cognitive ability while performing a specific action that relates to the safety of the user.

Still another control output 409 can be to utilize a communication interface 419, wherein the methods and systems can classify the user's brain signals into a mental command, to generate communications output that can be transmitted, received, and decoded by other communications equipment, such as mobile phone, computer monitor, and the like. The generated communications output can be a text, manufactured speech, images, or any other type of conventional communication format.

**(Features)**

Accordingly, aspects of the invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the invention may take the form of a computer program product embodied in one or more computer readable medium(s) having computer readable program code embodied thereon.

The present invention may be a system, a method, and/or a computer program product. The computer program product may include a computer readable storage medium (or media) having computer readable program instructions thereon for causing a processor to carry out aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

FIG. 5A is a schematic illustrating by non-limiting example a computing apparatus 500 that can be used to implement some techniques of the methods and systems, according to embodiments of the present disclosure. The computing apparatus or device 500 represents various forms of digital computers, such as laptops, desktops, workstations, personal digital assistants, servers, blade servers, mainframes, and other appropriate computers.

The computing device 500 can include a power source 508, a processor 509, a memory 510, a storage device 511, all connected to a bus 550. Further, a high-speed interface 512, a low-speed interface 513, high-speed expansion ports 514 and low speed expansion ports 515, can be connected to the bus 550. Also, a low-speed connection port 516 is in connection with the bus 550. Contemplated are various component configurations that may be mounted on a common motherboard depending upon the specific application. Further still, an input interface 517 can be connected via bus 550 to an external receiver 506 and an output interface 518. A receiver 519 can be connected to an external transmitter 507 and a transmitter 520 via the bus 550. Also connected to the bus 550 can be an external memory 504, external sensors 503, machine(s) 502 and an environment 501. Further, one or more external input/output devices 505 can be connected to the bus 550. A network interface controller (NIC) 521 can be adapted to connect through the bus 550 to a network 522, wherein data or other data, among other things, can be rendered on a third party display device, third party imaging device, and/or third party printing device outside of the computer device 500.

Contemplated is that the memory 510 can store instructions that are executable by the computer device 500, historical data, and any data that can be utilized by the methods and systems of the present disclosure. The memory 510 can include random access memory (RAM), read only memory (ROM), flash memory, or any other suitable memory systems. The memory 510 can be a volatile memory unit or units, and/or a non-volatile memory unit or units. The memory 510 may also be another form of computer-readable medium, such as a magnetic or optical disk.

Still referring to FIG. 5A, a storage device 511 can be adapted to store supplementary data and/or software modules used by the computer device 500. For example, the storage device 511 can store historical data and other related data as mentioned above regarding the present disclosure. Additionally, or alternatively, the storage device 511 can store historical data similar to data as mentioned above regarding the present disclosure. The storage device 511 can include a hard drive, an optical drive, a thumb-drive, an array of drives, or any combinations thereof. Further, the storage device 511 can contain a computer-readable medium, such as a floppy disk device, a hard disk device, an optical disk device, or a tape device, a flash memory or other similar solid state memory device, or an array of devices, including devices in a storage area network or other configurations. Instructions can be stored in an information carrier. The instructions, when executed by one or more processing devices (for example, processor 509), perform one or more methods, such as those described above.

The storage device 511 can be a computer readable storage medium that can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

The system can be linked through the bus 550 optionally to a display interface or user Interface (HMI) 523 adapted to connect the system to a display device 525 and keyboard 524, wherein the display device 525 can include a computer monitor, camera, television, projector, or mobile device, among others.

Still referring to FIG. 5A, the computer device 500 can include a user input interface 517 adapted to a printer interface (not shown) can also be connected through bus 550 and adapted to connect to a printing device (not shown), wherein the printing device can include a liquid inkjet printer, solid ink printer, large-scale commercial printer, thermal printer, UV printer, or dye-sublimation printer, among others.

The high-speed interface 512 manages bandwidth-intensive operations for the computing device 500, while the low-speed interface 513 manages lower bandwidth-intensive operations. Such allocation of functions is an example only. In some implementations, the high-speed interface 512 can be coupled to the memory 510, a user interface (HMI) 523, and to a keyboard 524 and display 525 (e.g., through a graphics processor or accelerator), and to the high-speed expansion ports 514, which may accept various expansion cards (not shown) via bus 550. In the implementation, the low-speed interface 513 is coupled to the storage device 511 and the low-speed expansion port 515, via bus 550. The low-speed expansion port 515, which may include various communication ports (e.g., USB, Bluetooth, Ethernet, wireless Ethernet) may be coupled to one or more input/output devices 505, and other devices a keyboard 524, a pointing device (not shown), a scanner (not shown), or a networking device such as a switch or router, e.g., through a network adapter.

Still referring to FIG. 5A, the computing device 500 may be implemented in a number of different forms, as shown in the figure. For example, it may be implemented as a standard server 526, or multiple times in a group of such servers. In addition, it may be implemented in a personal computer such as a laptop computer 527. It may also be implemented as part of a rack server system 528. Alternatively, components from the computing device 500 may be combined with other components in a mobile device (not shown), such as a mobile computing device 599 of FIG. 5B. Each of such devices may contain one or more of the computing device 500 and the mobile computing device 599 of FIG. 5B, and an entire system may be made up of multiple computing devices communicating with each other.

FIG. 5B is a schematic illustrating a travel computing apparatus that can be used to implement some techniques of the methods and systems, according to embodiments of the present disclosure. The travel computing apparatus or device 599 is intended to represent various forms of mobile devices, such as personal digital assistants, cellular telephones, smart-phones, and other similar computing devices. The travel computing device 599 may communicate wirelessly through the communication interface 564, which may include digital signal processing circuitry where necessary. The communication interface 564 may provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication may occur, for example, through the transceiver 571 using a radio-frequency. In addition, short-range communication may occur, such as using a Bluetooth, WiFi, or other such transceiver (not shown). In addition, a GPS (Global Positioning System) receiver module 573 may provide additional navigation and location related wireless data to the mobile computing device 599, which may be used as appropriate by applications running on the mobile computing device 599.

The travel computing device 599 includes a bus 595 connecting a processor 561, a memory 562, an input/output device 563, a communication interface 564, among other components. The bus 595 can also be connected to a storage device 565, such as a micro-drive or other device, to provide additional storage.

Referring to FIG. 5B, the processor 561 can execute instructions within the travel computing device 599, including instructions stored in the memory 562. The processor 561 may be implemented as a chipset of chips that include separate and multiple analog and digital processors. The processor 561 may provide, for example, for coordination of the other components of the travel computing device 599, such as control of user interfaces, applications run by the travel computing device 599, and wireless communication by the travel computing device 599.

The processor 561 may communicate with a user through a control interface 566 and a display interface 567 coupled to the display 568. The display 568 may be, for example, a TFT (Thin-Film-Transistor), Liquid Crystal Display or an OLED (Organic Light Emitting Diode) display, or other appropriate display technology. The display interface 567 may comprise appropriate circuitry for driving the display 568 to present graphical and other information to a user. The control interface 566 may receive commands from a user and convert them for submission to the processor 561. In addition, an external interface 569 may provide communication with the processor 561, so as to enable near area communication of the travel computing device 599 with other devices. The external interface 569 may provide, for example, for wired communication in some implementations, or for wireless communication in other implementations, and multiple interfaces may also be used.

Still referring to FIG. 5B, the memory 562 stores information within the travel computing device 599. The memory 562 can be implemented as one or more of a computer-readable medium or media, a volatile memory unit or units, or a non-volatile memory unit or units. An expansion memory 570 may also be provided and connected to the travel computing device 599 through an expansion interface 569, which may include, for example, a SIMM (single in line memory module) card interface. The expansion memory 570 may provide extra storage space for the travel computing device 599, or may also store applications or other information for the travel computing device 599. Specifically, the expansion memory 570 may include instructions to carry out or supplement the processes described above, and may include secure information also. Thus, for example, the expansion memory 570 may be providing as a security module for the mobile computing device 599, and may be programmed with instructions that permit secure use of the travel computing device 599. In addition, secure applications may be provided via the SIMM cards, along with additional information, such as placing identifying information on the SIMM card in a non-hackable manner.

The memory 562 may include, for example, flash memory and/or NVRAM memory (non-volatile random access memory), as discussed below. In some implementations, instructions are stored in an information carrier, that the instructions, when executed by one or more processing devices (for example, processor 561), perform one or more methods, such as those described above. The instructions can also be stored by one or more storage devices, such as one or more computer or machine readable mediums (for example, the memory 561, the expansion memory 570, or memory on the processor 562). In some implementations, the instructions can be received in a propagated signal, for example, over the transceiver 571 or the external interface 569.

The travel computing device 599 may also communicate audibly using an audio codec 572, which may receive spoken information from a user and convert it to usable digital information. The audio codec 572 may likewise generate audible sound for a user, such as through a speaker, e.g., in a handset of the travel computing device 599. Such sound may include sound from voice telephone calls, may include recorded sound (e.g., voice messages, music files, etc.) and may also include sound generated by applications operating on the travel computing device 599.

Still referring to FIG. 5B, the travel computing device 599 may be implemented in a number of different forms, as shown in the figure. For example, it may be implemented as a cellular telephone 574. It may also be implemented as part of a smart-phone 575, personal digital assistant, or other similar mobile device.

**(Definitions)**

As used herein, the term "neurophysiological data" also refers to brain imaging tools, including but not limited to CAT (computer-aided tomography) scans (otherwise known as CT or computed tomography) scans, PET (positron emission tomography) scans, magnetic resonance imaging (MRI) and functional magnetic resonance imaging (fMRI), ultrasound and single photon emission computed tomography (SPECT).

A "brain-computer interface (BCI)" is a communication device that classifies brain activity and controls a device such as a spelling application, a neuroprosthesis, or a wheelchair. Non-invasive BCIs rely on the electroencephalogram (EEG) to record brain signals. Signal processing for the BCI comprises 1) preprocessing, 2) feature extraction, and 3) classification. In the *preprocessing,* signals are filtered in the spatial and/or spectral domain. Spatial filters create a linear mixture of existing signal channels. Then, the preprocessed data is further processed in the *feature extraction.* The interaction of spatially separated brain areas can be observed through functional or effective connectivity. Functional connectivity reflects statistically related activation of brain areas, while effective connectivity explains the causality of these observed dependencies.

"Brain signals" may be measured by EEG, ECoG, MEG, fMRI, and others. They may also be measured remotely or indirectly through peripheral nerve or muscle activity. Depending on the intended application, signals of interest may represent time-course events, spatially distributed patterns, or combinations of the two. These signals can be generally studied in correlation with behavioral activity in order to map the measured brain activity to a particular behavioral activity. For example, activity in a specific region of the brain during word reading may be used to determine involvement of that brain region in the word reading process. Measurable activity (electrical, metabolic, magnetic, etc.) can be removed from the micro-level dynamics going on in the brain; therefore, which can become difficult to discriminate meaningful activity from meaningless activity.

A "signature" is a pattern within a signal or data stream that may be associated with a condition of interest in the signal generating system. Applications for brain activity signature detection and discrimination, can for example, be signals indicating various states or conditions such as: sleep, epilepsy, anxiety, degrees of anesthesia, and degrees of attention. Signals may also indicate the occurrence-or impending occurrence-of an event, such as: moving an arm, thinking of a specific idea, speaking, and so forth. Discerning signatures for such signals is useful in computer-brain interfacing applications and the like. Brain signals may also be used to identify their source, both in terms of the location within a particular individual's brain for mapping purposes and identification of one individual's brain signals, as differentiated from another's brain signals.

**(Features)**

An aspect of the present disclosure can include the sparse connectivity map is sparse in one or combination of a time domain and a spatial domain.

Another aspect of the present disclosure is that the processor enforces sparsity of the sparse connectivity map using a model relaying a time-varying neural current density responsible for electroencephalogram (EEG) potentials in the brain signals to a state of the brain. Such that the model is hierarchical VAR model with hierarchical lag structures, wherein the model is a VAR model subject to sparsity constraints, or that the model is a neural network.

An aspect of the present disclosure can be that the received brain signals are obtained from at least one sensor operable to sense signals indicative of the neurological activity, such that the at least one sensor is connected to the input interface. Such that the at least one sensor includes a transducer applied to the user to acquire electrical signals indicative of the neurological activity.

An aspect of the present disclosure can be that the device is a spelling application, a neuroprosthesis or a wheelchair. It is contemplated an aspect can be that the device is a car, a plane or a mechanical device operable by the user.

Another aspect of the present disclosure is that the device can be one of: a vehicle, with wheels, powered by an internal combustion engine and able to carry people and/or a load, such that the load produces revenue or does not produce revenue; a mechanical device such as a door with opening/closing abilities, that is operable by the BCI system by the user; an electronic device, such as a television, stereo, etc., that is operable by the BCI system by the user; and at least one device associated with everyday household activities by the user, such as coffee maker, dishwasher, turning on water of a faucet, hair dyer, light switches for a light, garage door switch for opening a garage door or activating an elevator system and on/off switch for a household related device.

**(Embodiments)**

The above description provides exemplary embodiments only, and is not intended to limit the scope, applicability, or configuration of the disclosure. Rather, the above description of the exemplary embodiments will provide those skilled in the art with an enabling description for implementing one or more exemplary embodiments. Contemplated are various changes that may be made in the function and arrangement of elements without departing from the scope of the subject matter disclosed as set forth in the appended claims.

Specific details are given in the above description to provide a thorough understanding of the embodiments. However, understood by one of ordinary skill in the art can be that the embodiments may be practiced without these specific details. For example, systems, processes, and other elements in the subject matter disclosed may be shown as components in block diagram form in order not to obscure the embodiments in unnecessary detail. In other instances, well-known processes, structures, and techniques may be shown without unnecessary detail in order to avoid obscuring the embodiments. Further, like reference numbers and designations in the various drawings indicated like elements.

Also, individual embodiments may be described as a process which is depicted as a flowchart, a flow diagram, a data flow diagram, a structure diagram, or a block diagram. Although a flowchart may describe the operations as a sequential process, many of the operations can be performed in parallel or concurrently. In addition, the order of the operations may be re-arranged. A process may be terminated when its operations are completed, but may have additional steps not discussed or included in a figure. Furthermore, not all operations in any particularly described process may occur in all embodiments. A process may correspond to a method, a function, a procedure, a subroutine, a subprogram, etc. When a process corresponds to a function, the function's termination can correspond to a return of the function to the calling function or the main function.

Furthermore, embodiments of the subject matter disclosed may be implemented, at least in part, either manually or automatically. Manual or automatic implementations may be executed, or at least assisted, through the use of machines, hardware, software, firmware, middleware, microcode, hardware description languages, or any combination thereof. When implemented in software, firmware, middleware or microcode, the program code or code segments to perform the necessary tasks may be stored in a machine readable medium. A processor(s) may perform the necessary tasks.

Further, embodiments of the present disclosure and the functional operations described in this specification can be implemented in digital electronic circuitry, in tangibly-embodied computer software or firmware, in computer hardware, including the structures disclosed in this specification and their structural equivalents, or in combinations of one or more of them. Further some embodiments of the present disclosure can be implemented as one or more computer programs, i.e.,

one or more modules of computer program instructions encoded on a tangible non transitory program carrier for execution by, or to control the operation of, data processing apparatus. Further still, program instructions can be encoded on an artificially generated propagated signal, e.g., a machine-generated electrical, optical, or electromagnetic signal, that is generated to encode information for transmission to suitable receiver apparatus for execution by a data processing apparatus. The computer storage medium can be a machine-readable storage device, a machine-readable storage substrate, a random or serial access memory device, or a combination of one or more of them.

According to embodiments of the present disclosure the term "data processing apparatus" can encompass all kinds of apparatus, devices, and machines for processing data, including by way of example a programmable processor, a computer, or multiple processors or computers. The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, or a combination of one or more of them.

A computer program (which may also be referred to or described as a program, software, a software application, a module, a software module, a script, or code) can be written in any form of programming language, including compiled or interpreted languages, or declarative or procedural languages, and it can be deployed in any form, including as a stand-alone program or as a module, component, subroutine, or other unit suitable for use in a computing environment. A computer program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a communication network. Computers suitable for the execution of a computer program include, by way of example, can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), e.g., the Internet.

## Claims

**1.** A brain-computer interface (BCI) system (100B, 500A, 500B) for actuating a control action, the brain-computer interface (BCI) system (100B, 500A, 500B) correlating brain activity of a user to a predetermined physiological response, the brain-computer interface (BCI) system (100B, 500A, 500B) comprising:

an input interface (9, 517) to receive brain signals indicative of an activity of a brain of the user;
an encoder (11) to encode the received brain signals to produce a sparse connectivity map of correlations among active regions of the brain, wherein the sparse connectivity map specifies more zero correlations than non-zero correlations between various active regions of the brain;
a classifier to classify the produced sparse connectivity map as an intended predetermined physiological response intended by the user; and
a controller (19) to communicate a control signal for actuating the control action according to the intended predetermined physiological response to a device (21).

2. The BCI system (100B, 500A, 500B) of claim 1, wherein the sparse connectivity map is sparse in a time domain and a spatial domain.

3. The BCI system (100B, 500A, 500B) of claim 1, wherein it enforces sparsity of the sparse connectivity map using a model relaying a time-varying neural current density responsible for electroencephalogram (EEG) potentials in the brain signals to a state of the brain.

4. The BCI system (100B, 500A, 500B) of claim 3, wherein the model is one of a hierarchical Vector Autoregression (VAR) model with hierarchical lag structures or a VAR model subject to sparsity constraints.

5. The BCI system (100B, 500A, 500B) of claim 1, wherein the device is one of a physical actuator controller (411) in communication with the user, a computer input device (413) in communication with a computer (423), a device guidance control (415) in communication with a vehicle (427) or a wheel chair (32, 429), a brain state monitoring device (417) in communication with the user or a communication interface (419) in communication with a mobile communication device (433) or a monitor (435) to another computer device.

6. The BCI system (100B, 500A, 500B) of claim 3, wherein the model is a neural network.

7. The BCI system (100B, 500A, 500B) of claim 1, wherein the received brain signals are obtained from at least one sensor (503) operable to sense signals indicative of the neurological activity, whereby the at least one sensor (503) is connected to the input interface (9, 517).

8. The BCI system (100B, 500A, 500B) of claim 7, wherein the at least one sensor (503) includes a transducer applied to the user to acquire electrical signals indicative of the neurological activity.

9. The BCI system (100B, 500A, 500B) of claim 7, wherein the device is one of a spelling application, a neuroprosthesis or a wheelchair (32, 429).

10. The system (100B, 500A, 500B) of claim 1, wherein the device has an interface, wherein the interface is a device guidance control for a heating, ventilation and air conditioning (HVAC) system (31, 425).

11. A method of analysis for correlating brain activity of a user to a predetermined physiological response, comprising:

using an input interface (9, 517) to receive brain signals indicative of an activity of a brain of the user;
using an encoder (11) for encoding the received brain signals to produce a sparse connectivity map of correlations among active regions of the brain, wherein the sparse connectivity map specifies more zero correlations than non-zero correlations between various active regions of the brain;
using a classifier to classify the sparse connectivity map as an intended predetermined physiological response intended by the user; and
using a controller (19) to communicate a control signal for actuating the control action according to the intended predetermined physiological response to a device.

## Patentansprüche

1. Gehirn-Computer-Schnittstellen-(BCI)-System (100B, 500A, 500B) zum Auslösen einer Steueraktion, wobei das Gehirn-Computer-Schnittstellen-(BCI)-System (100B, 500A, 500B)
eine Gehirnaktivität eines Benutzers mit einer vorbestimmten physiologischen Reaktion korreliert, wobei das Gehirn-Computer-Schnittstellen-(BCI)-System (100B, 500A, 500B) umfasst:

eine Eingabeschnittstelle (9, 517), um Gehirnsignale, die eine Aktivität des Gehirns des Benutzers anzeigen, zu empfangen;

einen Codierer (11), um die empfangenen Gehirnsignale zu codieren, um eine dünnbesetzte Konnektivitätskarte von Korrelationen zwischen aktiven Regionen des Gehirns zu erzeugen, wobei die dünnbesetzte Konnektivitätskarte mehr Null-Korrelationen als Nicht-Null-Korrelationen zwischen verschiedenen aktiven Regionen des Gehirns spezifiziert;
einen Klassifikator, um die erzeugte dünnbesetzte Konnektivitätskarte als eine beabsichtigte vorbestimmte physiologische Reaktion, die vom Benutzer beabsichtigt ist, zu klassifizieren; und
ein Steuereinheit (19), um ein Steuersignal zur Auslösung der Steueraktion gemäß der beabsichtigten vorbestimmten physiologischen Reaktion an eine Einrichtung (21) zu übermitteln.

**2.** BCI-System (100B, 500A, 500B) nach Anspruch 1, wobei die dünnbesetzte Konnektivitätskarte in einer Zeitdomäne und einer Raumdomäne dünnbesetzt ist.

**3.** BCI-System (100B, 500A, 500B) nach Anspruch 1, wobei es die Dünnbesetztheit der dünnbesetzten Konnektivitätskarte mit Hilfe eines Modells erzwingt, das eine zeitlich veränderliche neuronale Stromdichte, die für Elektroenzephalogramm (EEG)-Potenziale in den Gehirnsignalen verantwortlich ist, auf einen Zustand des Gehirns überträgt.

**4.** BCI-System (100B, 500A, 500B) nach Anspruch 3, wobei das Modell entweder ein hierarchisches Vektor-Autoregressions-(VAR)-Modell mit hierarchischen Lag-Strukturen oder ein VAR-Modell ist, das Dünnbesetztheits-Randbedingungen unterliegt.

**5.** BCI-System (100B, 500A, 500B) nach Anspruch 1, wobei es sich bei der Einrichtung entweder um eine physische Aktuator-Steuereinheit (411) in Kommunikation mit dem Benutzer, eine Computereingabeeinrichtung (413) in Kommunikation mit einem Computer (423), eine Einrichtungsleitsteuerung (415) in Kommunikation mit einem Fahrzeug (427) oder einem Rollstuhl (32, 429), eine Gehirnzustandsüberwachungseinrichtung (417) in Kommunikation mit dem Benutzer oder eine Kommunikationsschnittstelle (419) in Kommunikation mit einer mobilen Kommunikationseinrichtung (433) oder einem Monitor (435) zu einer anderen Computereinrichtung handelt.

**6.** BCI-System (100B, 500A, 500B) nach Anspruch 3, wobei das Modell ein neuronales Netz ist.

**7.** BCI-System (100B, 500A, 500B) nach Anspruch 1, wobei die empfangenen Gehirnsignale von mindestens einem Sensor (503) erhalten werden, der in der Lage ist, Signale zu erfassen, welche die neurologische Aktivität anzeigen, wobei der mindestens eine Sensor (503) mit der Eingabeschnittstelle (9, 517) verbunden ist.

**8.** BCI-System (100B, 500A, 500B) nach Anspruch 7, wobei der mindestens eine Sensor (503) einen Wandler umfasst, der an den Benutzer angelegt wird, um elektrische Signale zu erwerben, welche die neurologische Aktivität anzeigen.

**9.** BCI-System (100B, 500A, 500B) nach Anspruch 7, wobei es sich bei der Einrichtung um eine Buchstabieranwendung, eine Neuroprothese oder einen Rollstuhl handelt (32, 429).

**10.** System (100B, 500A, 500B) nach Anspruch 1, wobei die Einrichtung eine Benutzeroberfläche aufweist, wobei die Benutzeroberfläche eine Einrichtungsleitsteuerung für ein Erwärmungs-, Belüftungs- und Klimatisierungs-(HVAC)-System (31, 425) ist.

**11.** Analyseverfahren zum Korrelieren einer Gehirnaktivität eines Benutzers mit einer vorbestimmten physiologischen Reaktion, umfassend:

Nutzen einer Eingabeschnittstelle (9, 517), um Gehirnsignale, die eine Aktivität des Gehirns des Benutzers anzeigen, zu empfangen;
einen Codierer (11), um die empfangenen Gehirnsignale zu codieren, um eine dünnbesetzte Konnektivitätskarte von Korrelationen zwischen aktiven Regionen des Gehirns zu erzeugen, wobei die dünnbesetzte Konnektivitätskarte mehr Null-Korrelationen als Nicht-Null-Korrelationen zwischen verschiedenen aktiven Regionen des Gehirns spezifiziert;
Nutzen eines Klassifikators, um die dünnbesetzte Konnektivitätskarte als eine beabsichtigte vorbestimmte physiologische Reaktion, die von dem Benutzer beabsichtigt ist, zu klassifizieren; und
Nutzen einer Steuereinheit (19), um ein Steuersignal zur Auslösung der Steueraktion gemäß der beabsichtigten vorbestimmten physiologischen Reaktion an eine Einrichtung zu übermitteln.

## Revendications

**1.** Système d'interface cerveau-ordinateur (BCI) (100B, 500A, 500B) pour actionner une commande, le système d'interface cerveau-ordinateur (BCI) (100B, 500A, 500B)
corrélant l'activité cérébrale d'un utilisateur à une réponse physiologique prédéterminée, le système d'interface cerveau-ordinateur (BCI) (100B, 500A, 500B) comprenant :

une interface d'entrée (9, 517) pour recevoir des signaux cérébraux indiquant une activité du cerveau de l'utilisateur;
un codeur (11) pour coder les signaux cérébraux reçus afin de produire une carte de connectivité éparse des corrélations entre les régions actives du cerveau, dans lequel la carte de connectivité éparse spécifie plus de corrélations nulles que de corrélations non nulles entre les différentes régions actives du cerveau ;
un classificateur pour classer la carte de connectivité éparse produite comme une réponse physiologique prédéterminée voulue par l'utilisateur ; et
un dispositif de commande (19) pour communiquer à un dispositif (21) un signal de commande pour actionner la commande selon la réponse physiologique prédéterminée voulue.

**2.** Système BCI (100B, 500A, 500B) selon la revendication 1, dans lequel la carte de connectivité éparse est éparse dans un domaine temporel et un domaine spatial.

**3.** Système BCI (100B, 500A, 500B) selon la revendication 1, qui impose la sparsité de la carte de connectivité éparse en utilisant un modèle qui met en relation une densité de courant neuronal variable dans le temps, responsable de potentiels d'électroencéphalogramme (EEG) dans les signaux cérébraux, et l'état cérébral.

**4.** Système BCI (100B, 500A, 500B) selon la revendication 3, dans lequel le modèle est un modèle parmi un modèle d'autorégression vectorielle (VAR) hiérarchique avec des structures de retard hiérarchiques ou un modèle VAR soumis à des contraintes de sparsité.

**5.** Système BCI (100B, 500A, 500B) selon la revendication 1, dans lequel le dispositif est un dispositif parmi un actionneur physique (411) en communication avec l'utilisateur, un dispositif d'entrée informatique (413) en communication avec un ordinateur (423), un contrôle de guidage de dispositif (415) en communication avec un véhicule (427) ou un fauteuil roulant (32, 429), un dispositif de surveillance de l'état du cerveau (417) en communication avec l'utilisateur ou une interface de communication (419) en communication avec un dispositif de communication mobile (433) ou un moniteur (435) connecté à un autre dispositif informatique.

**6.** Système BCI (100B, 500A, 500B) selon la revendication 3, dans lequel le modèle est un réseau neuronal.

**7.** Système BCI (100B, 500A, 500B) selon la revendication 1, dans lequel les signaux cérébraux reçus sont obtenus à partir d'au moins un capteur (503) capable de détecter des signaux indiquant l'activité neurologique, moyennant quoi l'au moins un capteur (503) est connecté à l'interface d'entrée (9, 517).

**8.** Système BCI (100B, 500A, 500B) selon la revendication 7, dans lequel l'au moins un capteur (503) comprend un transducteur appliqué à l'utilisateur pour acquérir des signaux électriques indiquant l'activité neurologique.

**9.** Système BCI (100B, 500A, 500B) selon la revendication 7, dans lequel le dispositif est un dispositif parmi une application orthographique, une neuroprothèse ou un fauteuil roulant (32, 429).

**10.** Système BCI (100B, 500A, 500B) selon la revendication 1, dans lequel le dispositif comporte une interface, dans lequel l'interface est une commande de guidage de dispositif pour un système de chauffage, de ventilation et de climatisation (HVAC) (31, 425).

**11.** Procédé d'analyse pour corréler l'activité cérébrale d'un utilisateur à une réponse physiologique prédéterminée, comprenant :

l'utilisation d'une interface d'entrée (9, 517) pour recevoir des signaux cérébraux indiquant une activité du cerveau de l'utilisateur ;
l'utilisation d'un codeur (11) pour coder les signaux cérébraux reçus afin de produire une carte de connectivité éparse des corrélations entre les régions actives du cerveau, dans lequel la carte de connectivité éparse spécifie plus de corrélations nulles que de corrélations non nulles entre les différentes régions actives du cerveau ;
l'utilisation d'un classificateur pour classer la carte de connectivité éparse comme une réponse physiologique prédéterminée et voulue par l'utilisateur ; et
l'utilisation d'un dispositif de commande (19) pour communiquer à un dispositif un signal de commande pour actionner la commande selon la réponse physiologique prédéterminée voulue.

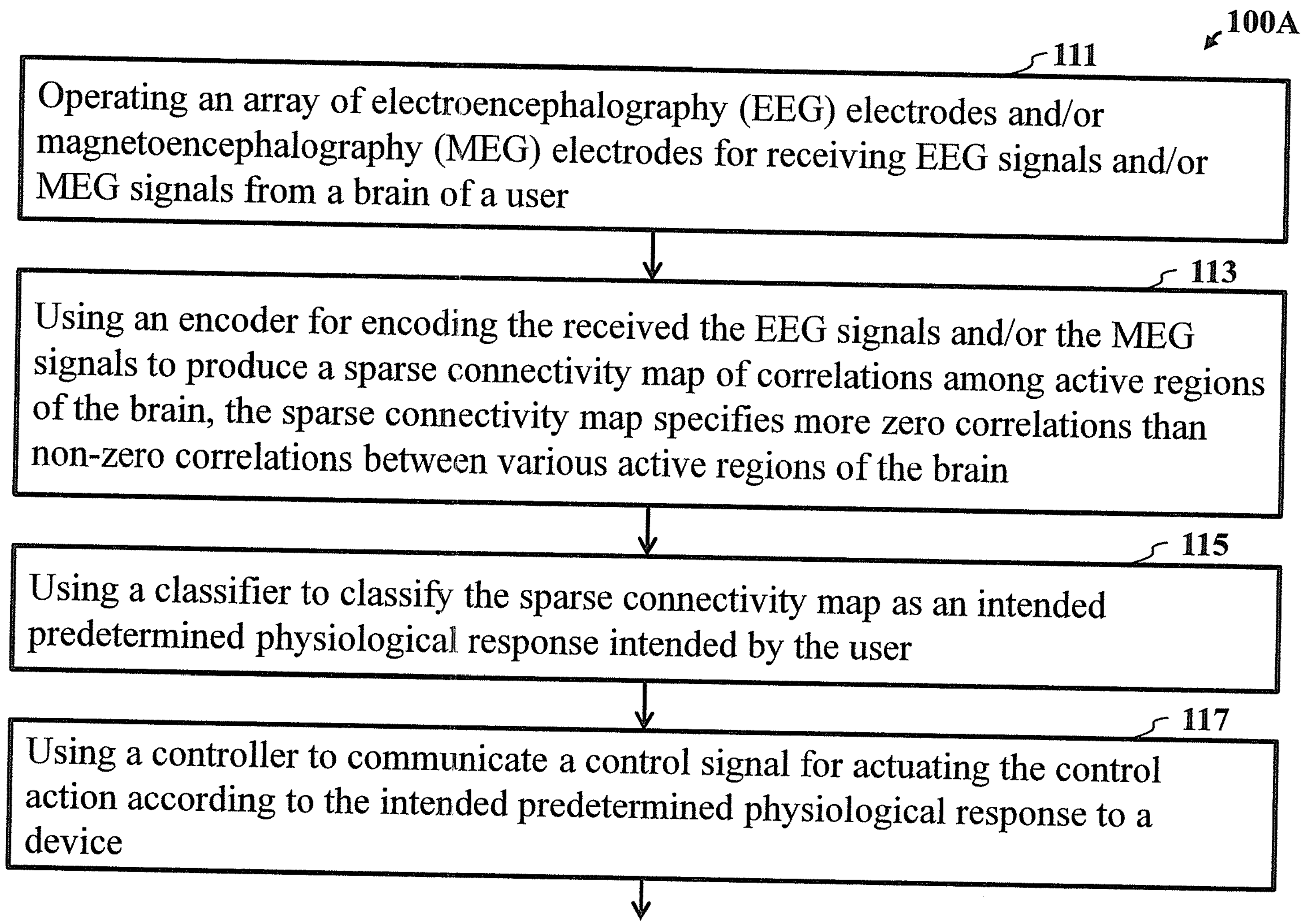
100A
111
Operating an array of electroencephalography (EEG) electrodes and/or magnetoencephalography (MEG) electrodes for receiving EEG signals and/or MEG signals from a brain of a user
113
Using an encoder for encoding the received the EEG signals and/or the MEG signals to produce a sparse connectivity map of correlations among active regions of the brain, the sparse connectivity map specifies more zero correlations than non-zero correlations between various active regions of the brain
115
Using a classifier to classify the sparse connectivity map as an intended predetermined physiological response intended by the user
117
Using a controller to communicate a control signal for actuating the control action according to the intended predetermined physiological response to a device

FIG. 1A

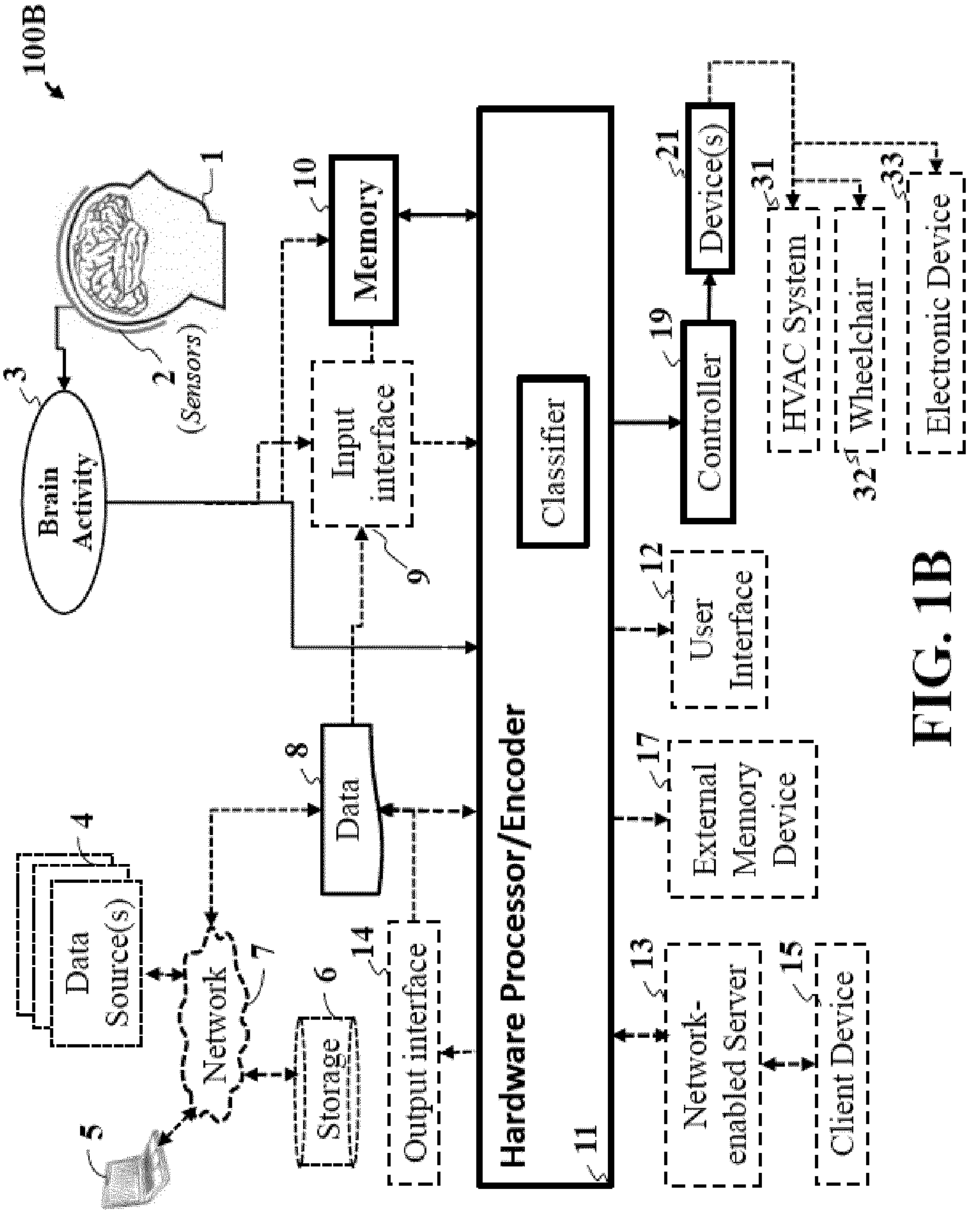
100B
1
2
(Sensors)
3
Brain Activity
4
Data Source(s)
5
Network
7
Storage
6
Output interface
14
Data
8
Input interface
9
Memory
10
Hardware Processor/Encoder
11
Classifier
Controller
19
Device(s)
21
HVAC System
31
Wheelchair
32
Electronic Device
33
User Interface
12
External Memory Device
17
Network-enabled Server
13
Client Device
15

FIG. 1B

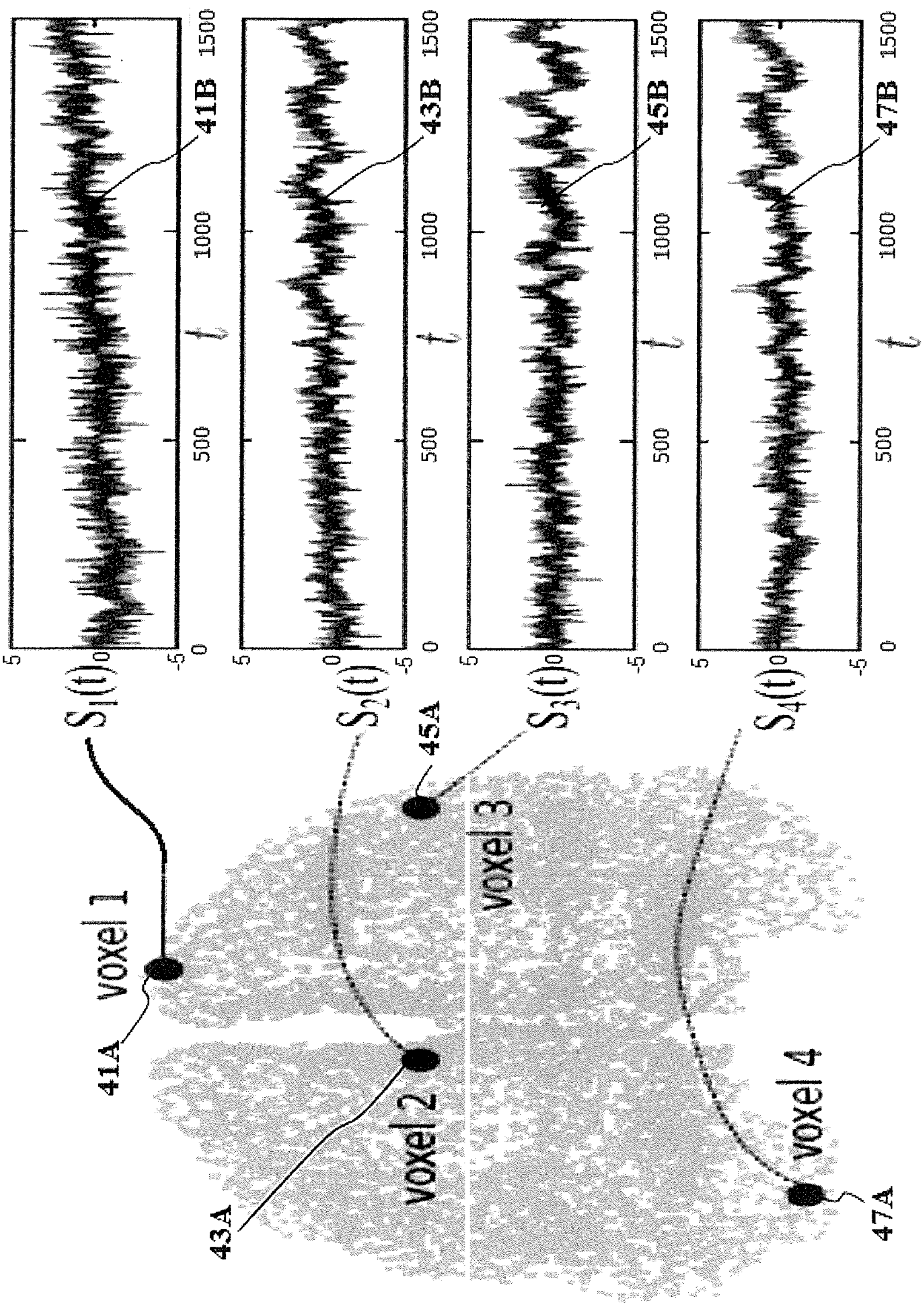
41A
voxel 1
43A
voxel 2
45A
voxel 3
47A
voxel 4
$S_1(t)$
$S_2(t)$
$S_3(t)$
$S_4(t)$
41B
43B
45B
47B
5
0
-5
0
500
1000
1500
t

FIG. 1C

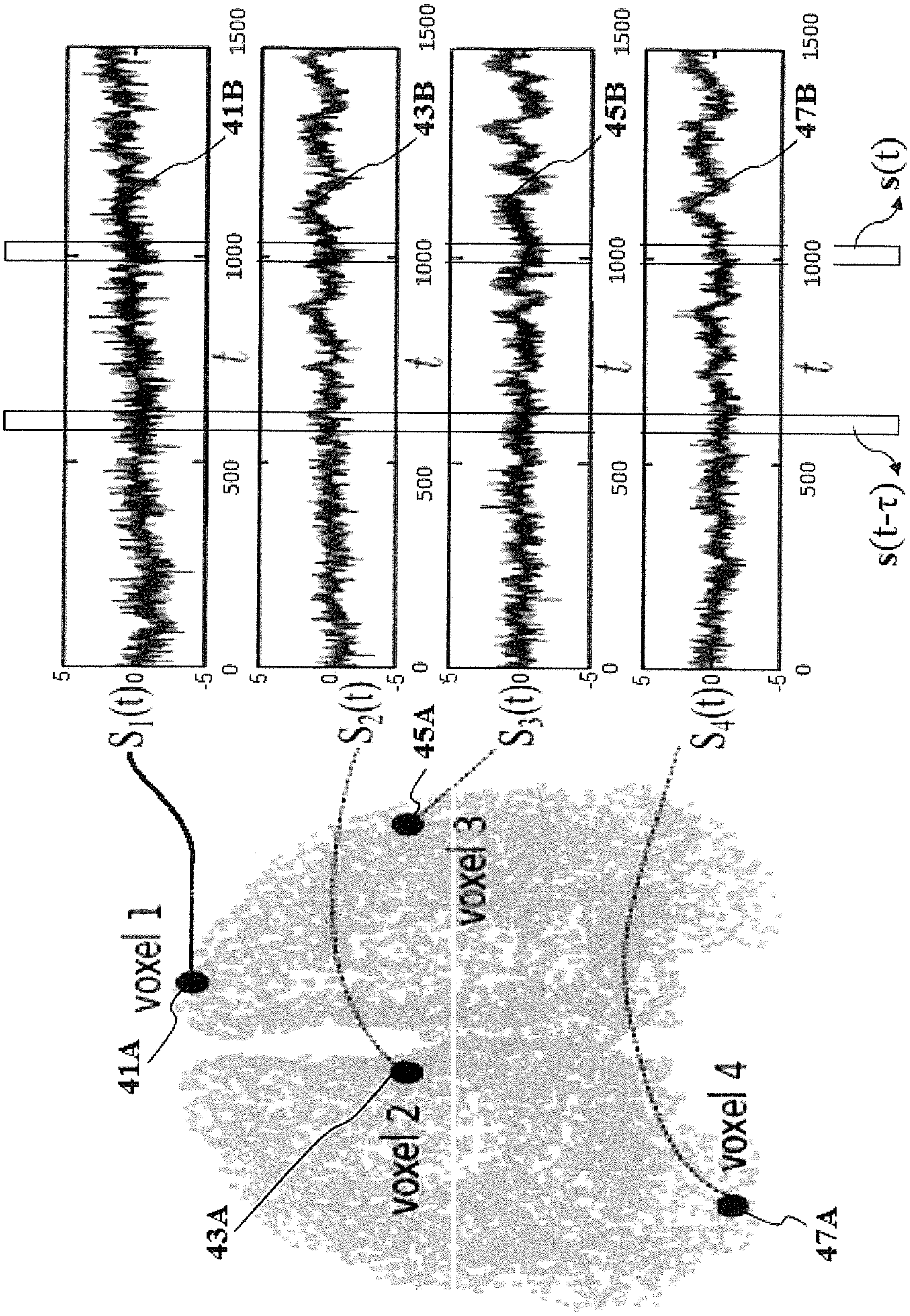
41A
voxel 1
43A
voxel 2
45A
voxel 3
47A
voxel 4
S1(t)
S2(t)
S3(t)
S4(t)
41B
43B
45B
47B
5
0
-5
0
500
1000
1500
t
s(t-τ)
s(t)

FIG. 1D

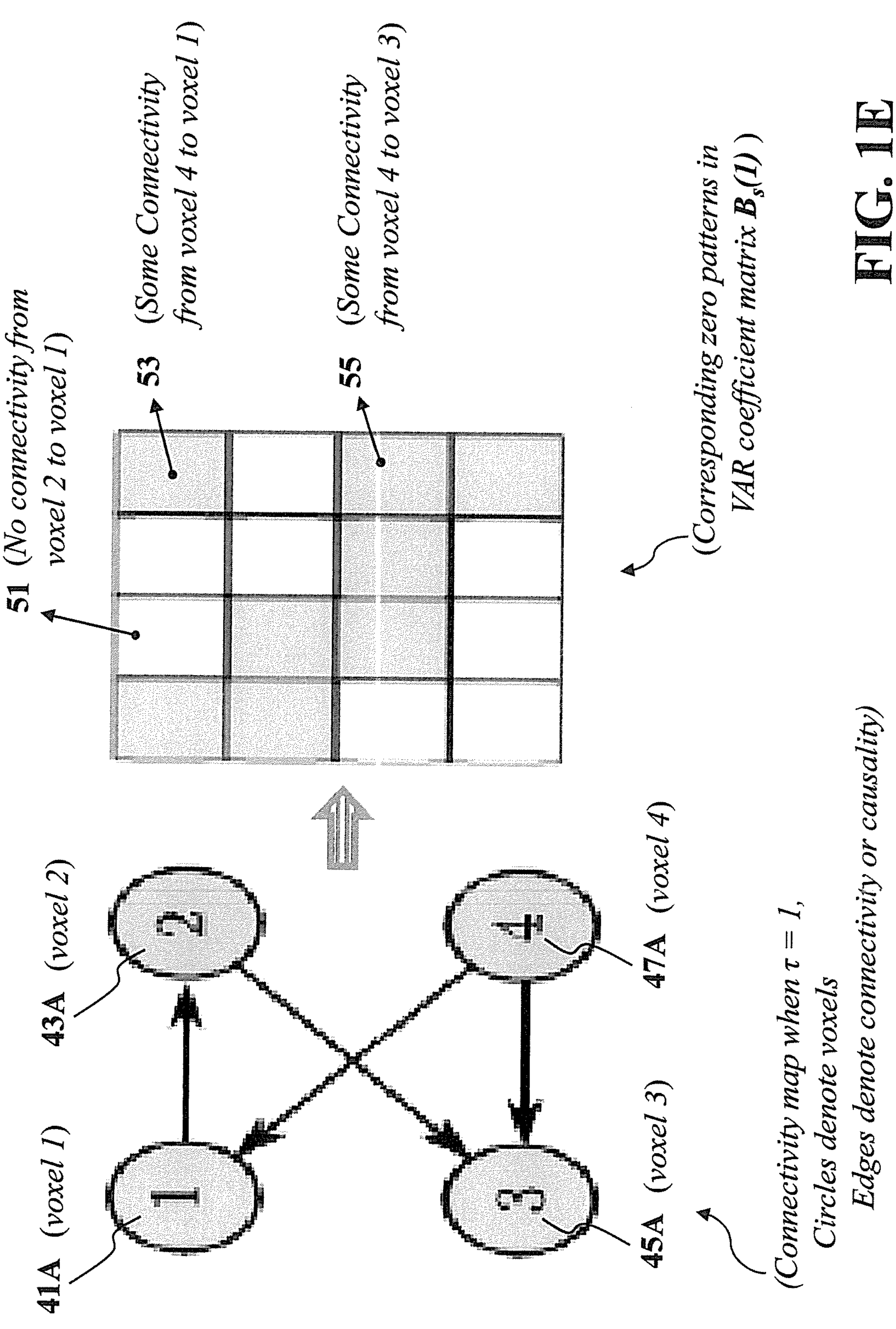
41A (voxel 1)
43A (voxel 2)
45A (voxel 3)
47A (voxel 4)
1
2
3
4
(Connectivity map when τ = 1,
Circles denote voxels
Edges denote connectivity or causality)
51 (No connectivity from voxel 2 to voxel 1)
53 (Some Connectivity from voxel 4 to voxel 1)
55 (Some Connectivity from voxel 4 to voxel 3)
(Corresponding zero patterns in VAR coefficient matrix Bs(1))

FIG. 1E

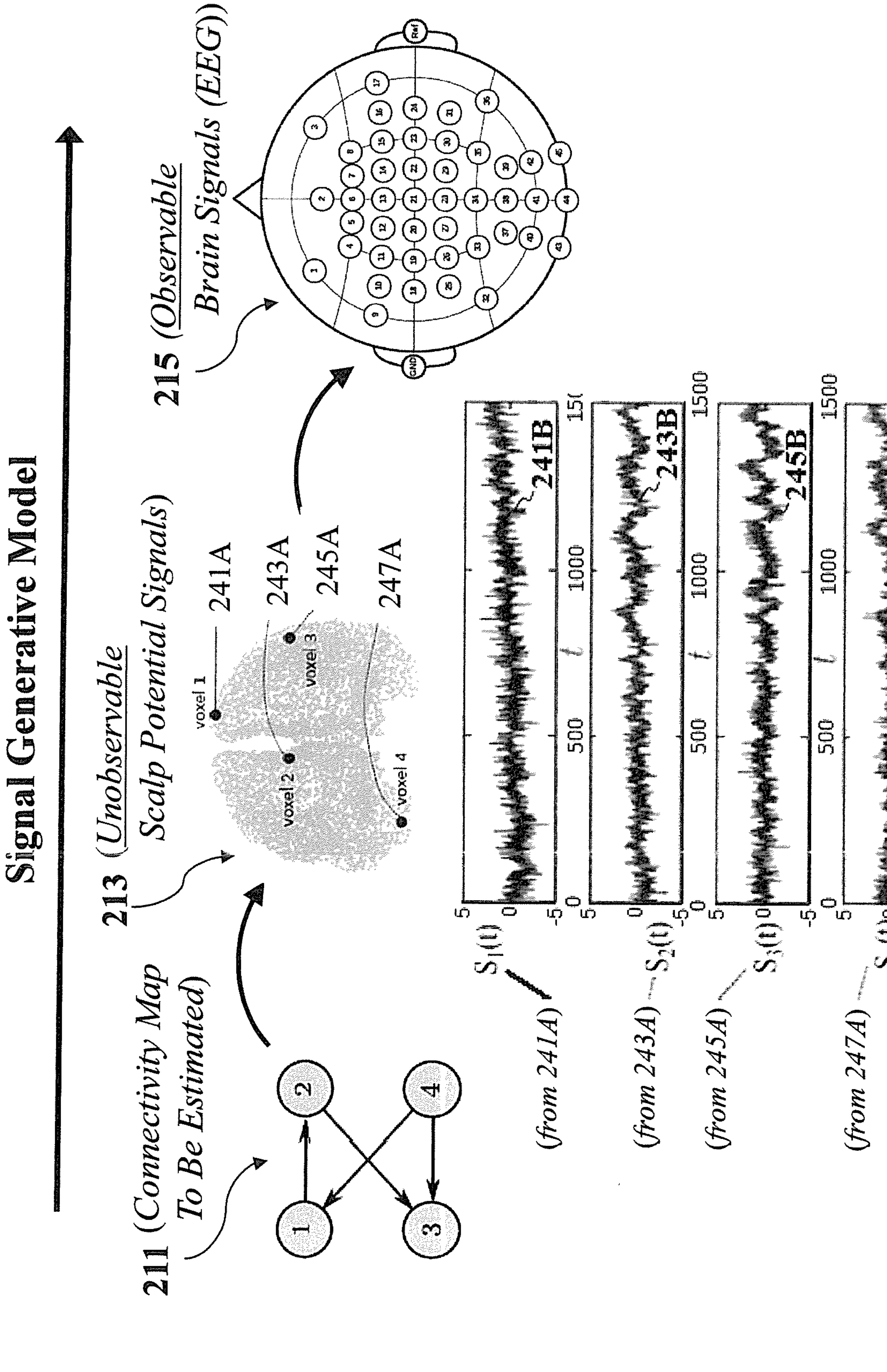
Signal Generative Model
211 (Connectivity Map
To Be Estimated)
213 (Unobservable
Scalp Potential Signals)
215 (Observable
Brain Signals (EEG))
241A
243A
245A
247A
voxel 1
voxel 2
voxel 3
voxel 4
$S_1(t)$
$S_2(t)$
$S_3(t)$
$S_4(t)$
(from 241A)
(from 243A)
(from 245A)
(from 247A)
241B
243B
245B
247B
t

FIG. 2A

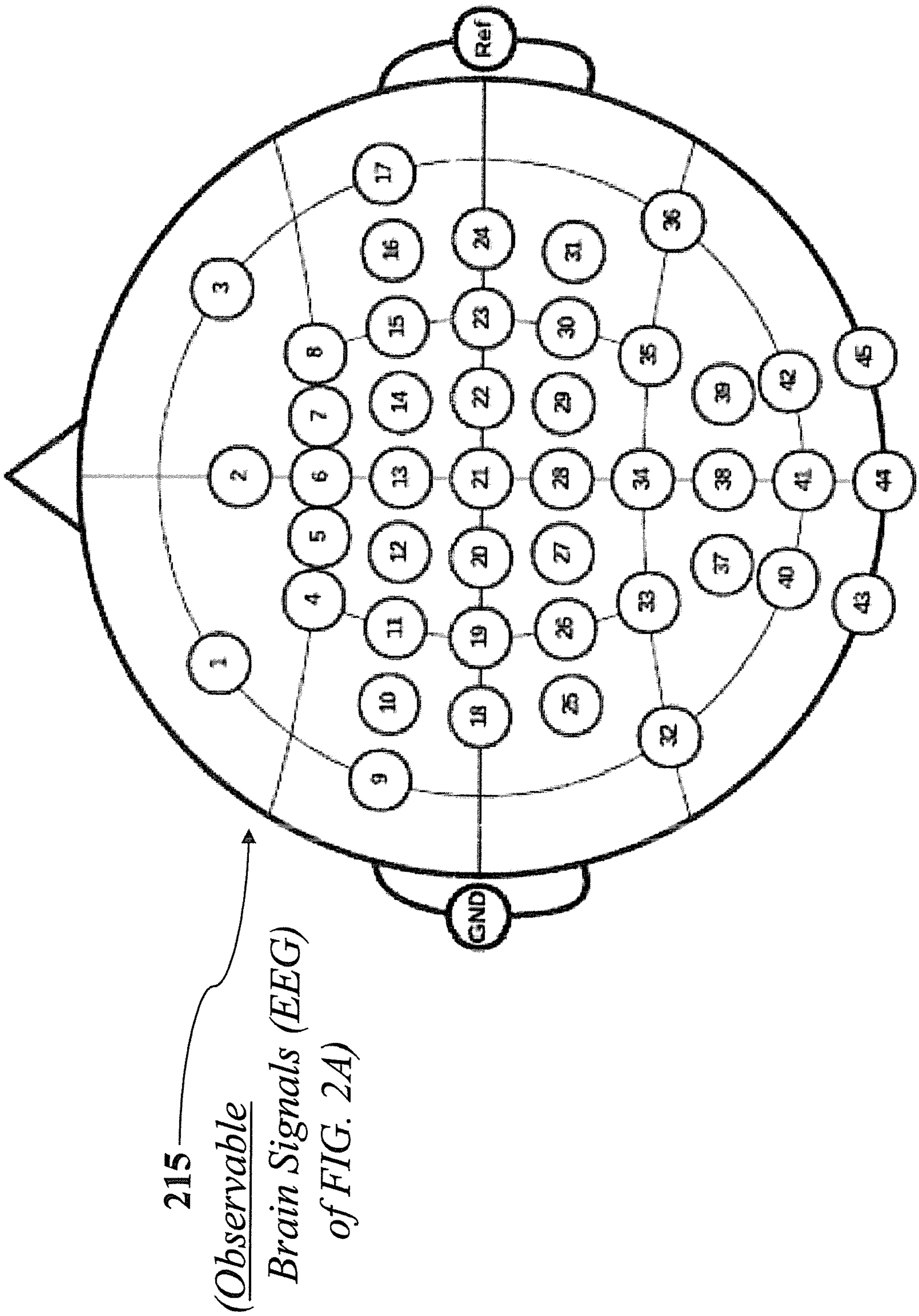
215
(Observable
Brain Signals (EEG)
of FIG. 2A)
Ref
GND
1
2
3
4
5
6
7
8
9
10
11
12
13
14
15
16
17
18
19
20
21
22
23
24
25
26
27
28
29
30
31
32
33
34
35
36
37
38
39
40
41
42
43
44
45

FIG. 2B

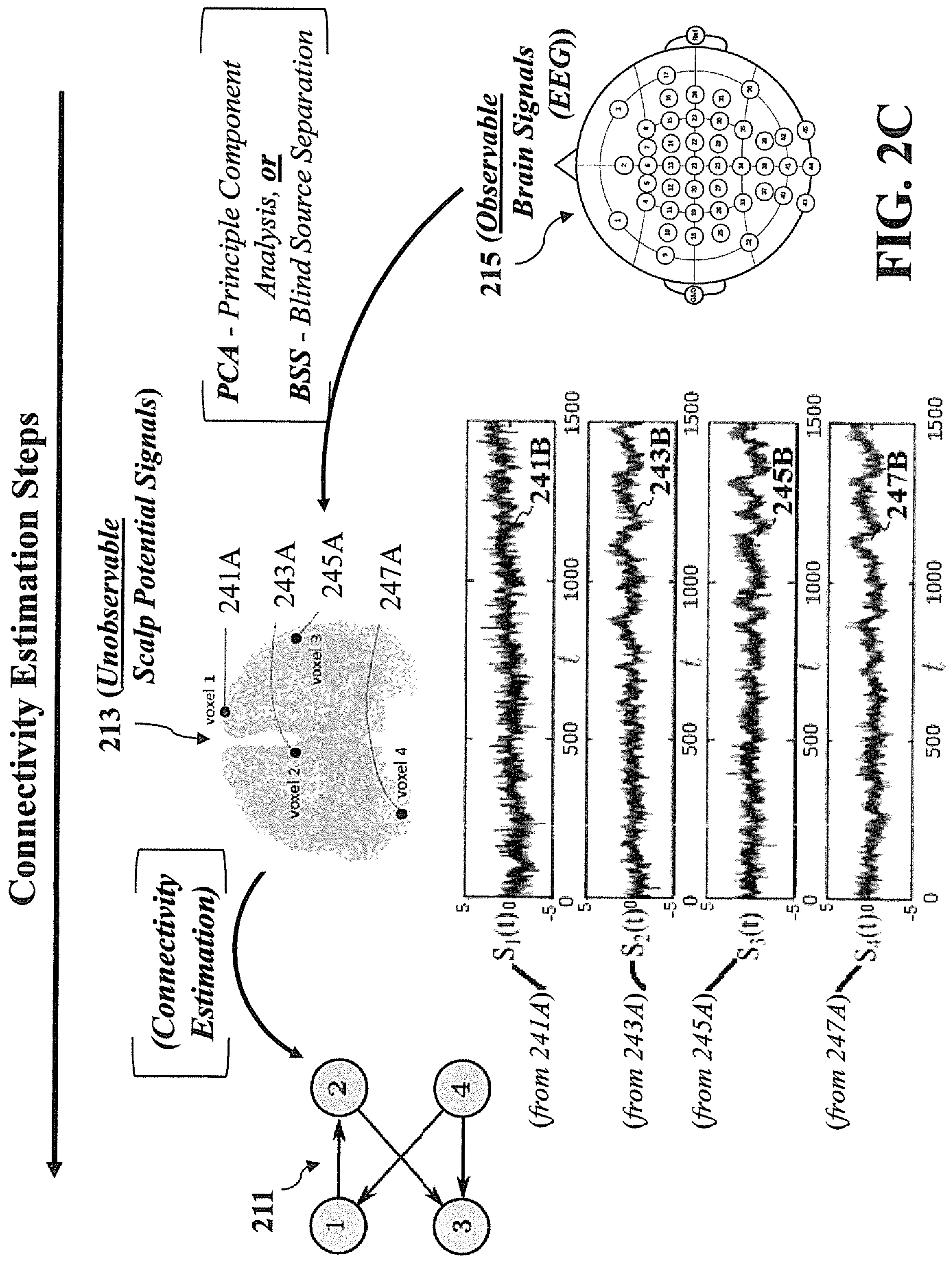

Connectivity Estimation Steps
211
1
2
3
4
(Connectivity Estimation)
213 (Unobservable Scalp Potential Signals)
voxel 1
voxel 2
voxel 3
voxel 4
241A
243A
245A
247A
PCA - Principle Component Analysis, or
BSS - Blind Source Separation
215 (Observable Brain Signals (EEG))
GND
Ref
(from 241A)
(from 243A)
(from 245A)
(from 247A)
$S_1(t)$
$S_2(t)$
$S_3(t)$
$S_4(t)$
241B
243B
245B
247B
5
0
-5
0
500
1000
1500
t


FIG. 2C

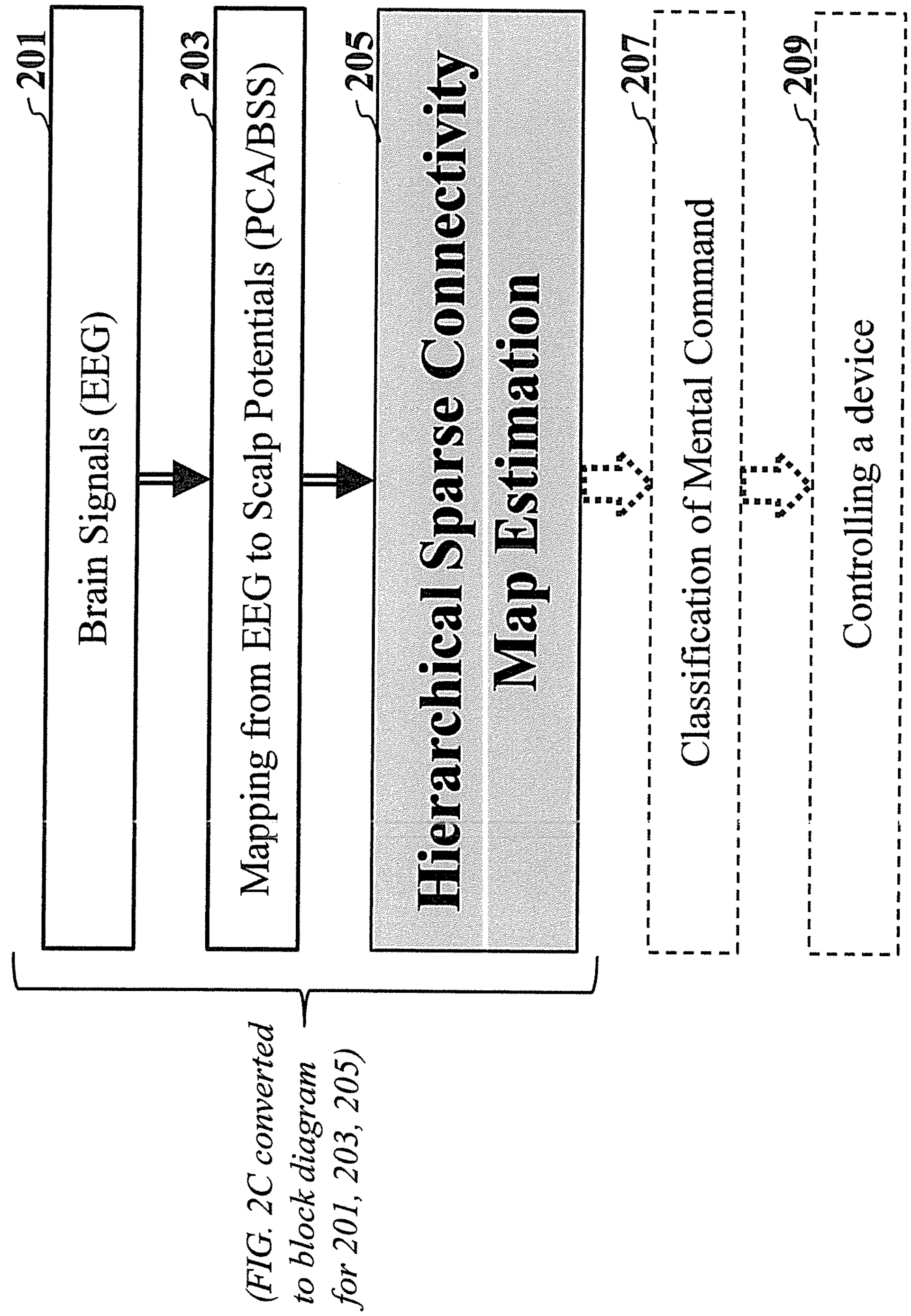
201
Brain Signals (EEG)
203
Mapping from EEG to Scalp Potentials (PCA/BSS)
205
Hierarchical Sparse Connectivity Map Estimation
207
Classification of Mental Command
209
Controlling a device
(FIG. 2C converted to block diagram for 201, 203, 205)

FIG. 2D

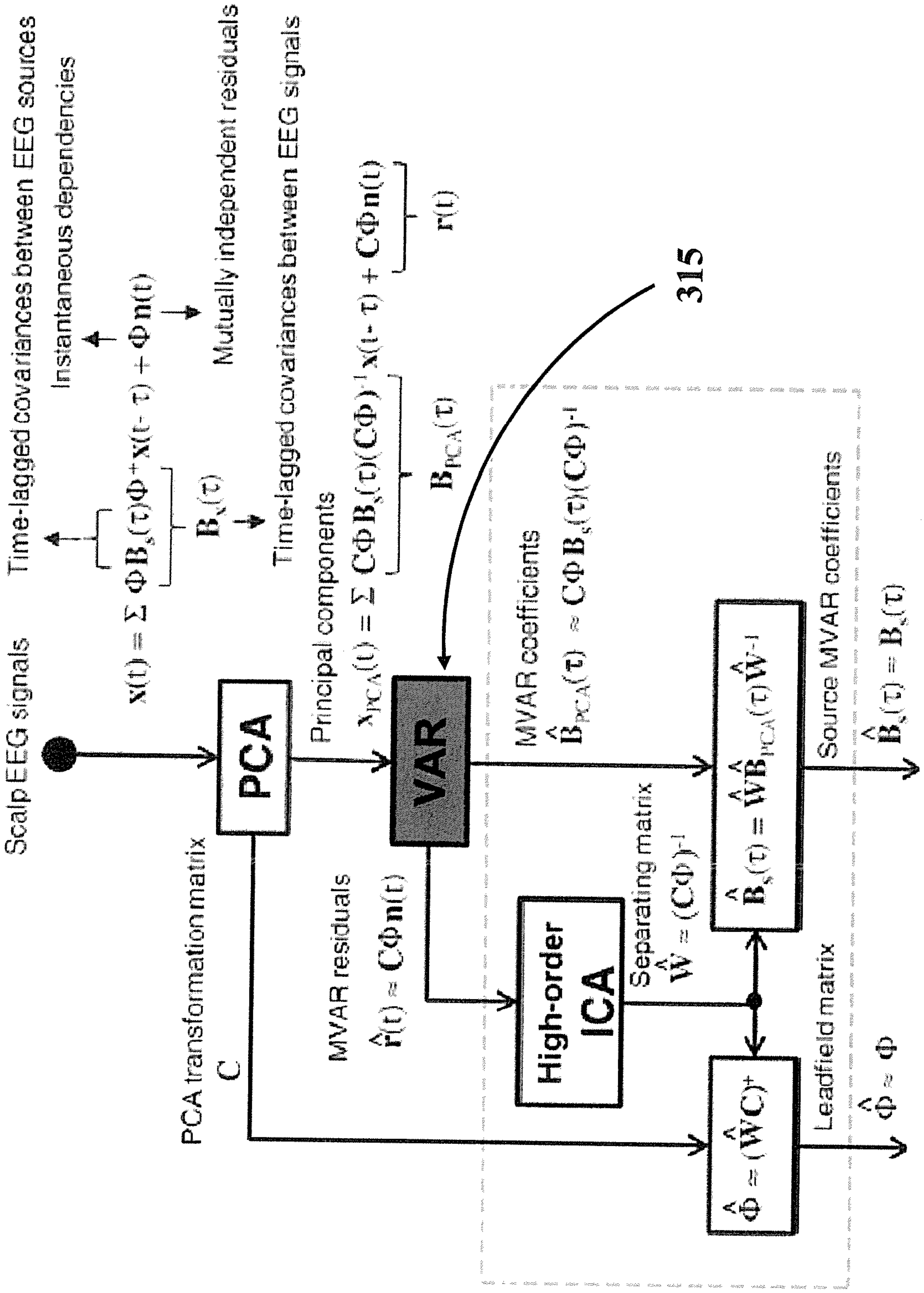
Scalp EEG signals
PCA
PCA transformation matrix
C
Principal components
VAR
MVAR residuals
High-order ICA
Separating matrix
MVAR coefficients
Source MVAR coefficients
Leadfield matrix
Source Localization
Connectivity
Time-lagged covariances between EEG sources
Instantaneous dependencies
Mutually independent residuals
Time-lagged covariances between EEG signals
315

FIG. 3A

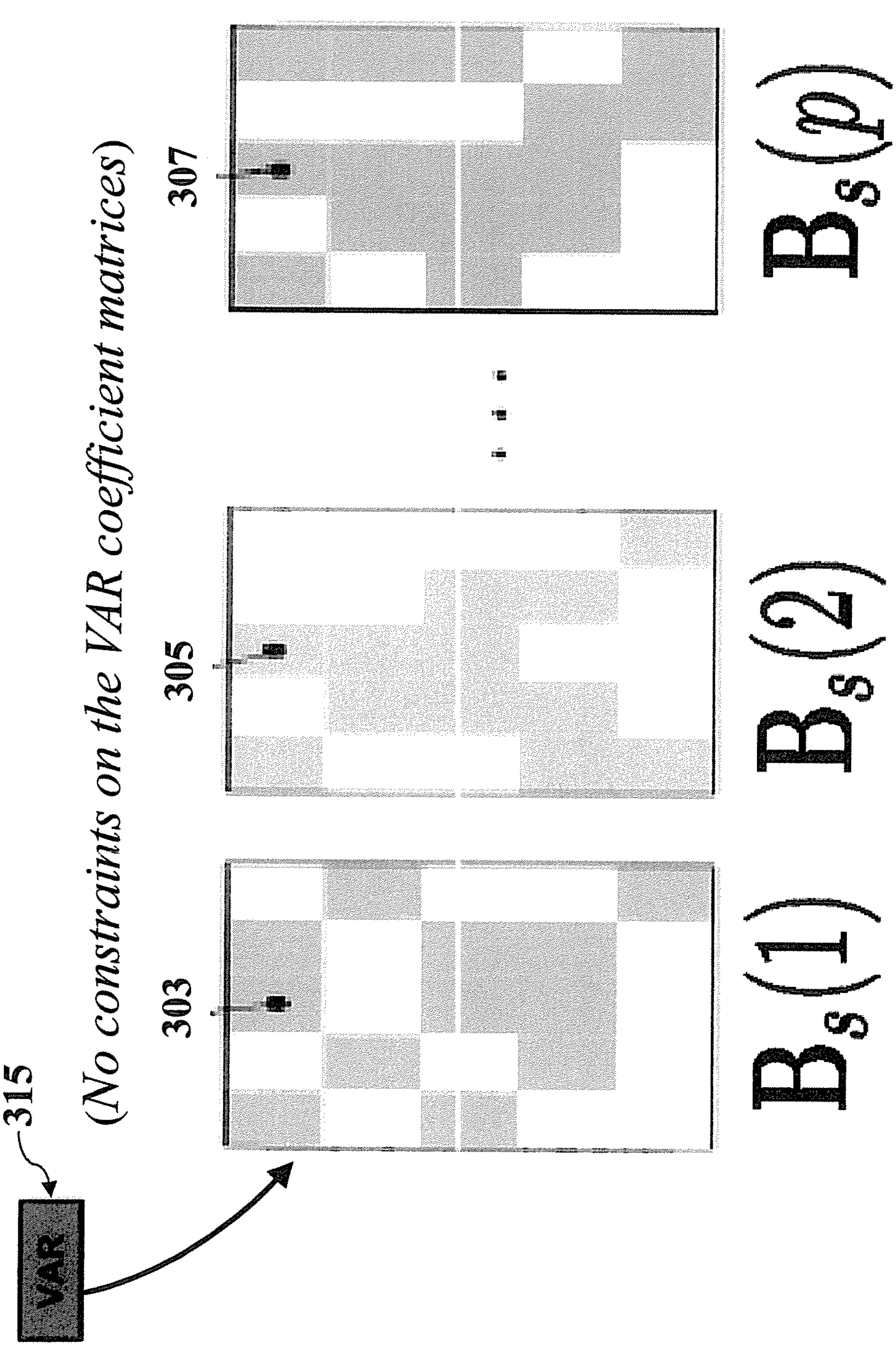
VAR
315
(No constraints on the VAR coefficient matrices)
303
305
307
$B_s(1)$
$B_s(2)$
$B_s(p)$

FIG. 3B

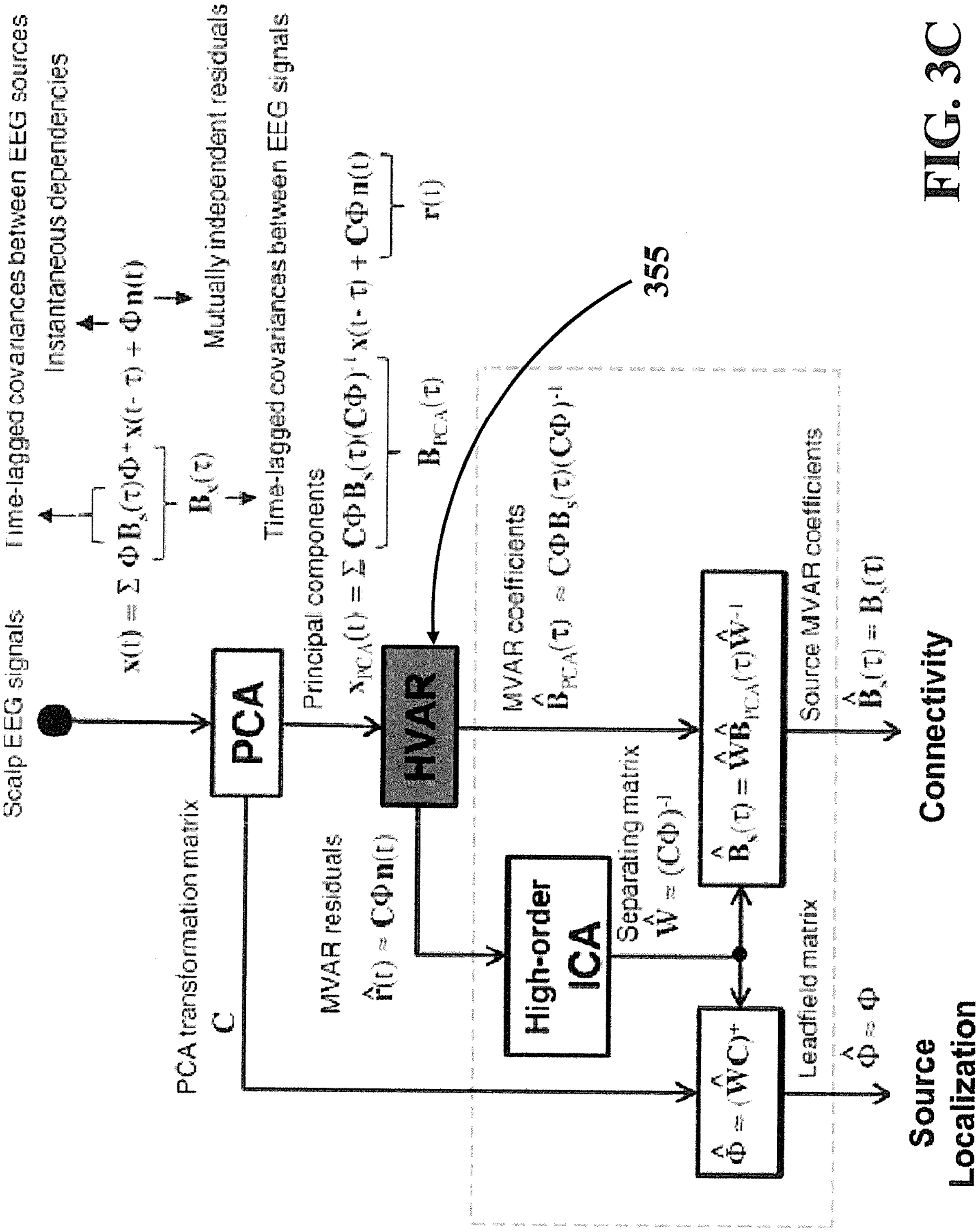
Scalp EEG signals
PCA
PCA transformation matrix
C
Principal components
HVAR
MVAR residuals
High-order ICA
Separating matrix
MVAR coefficients
Source MVAR coefficients
Leadfield matrix
Time-lagged covariances between EEG sources
Instantaneous dependencies
Mutually independent residuals
Time-lagged covariances between EEG signals
355
Connectivity
Source Localization

FIG. 3C

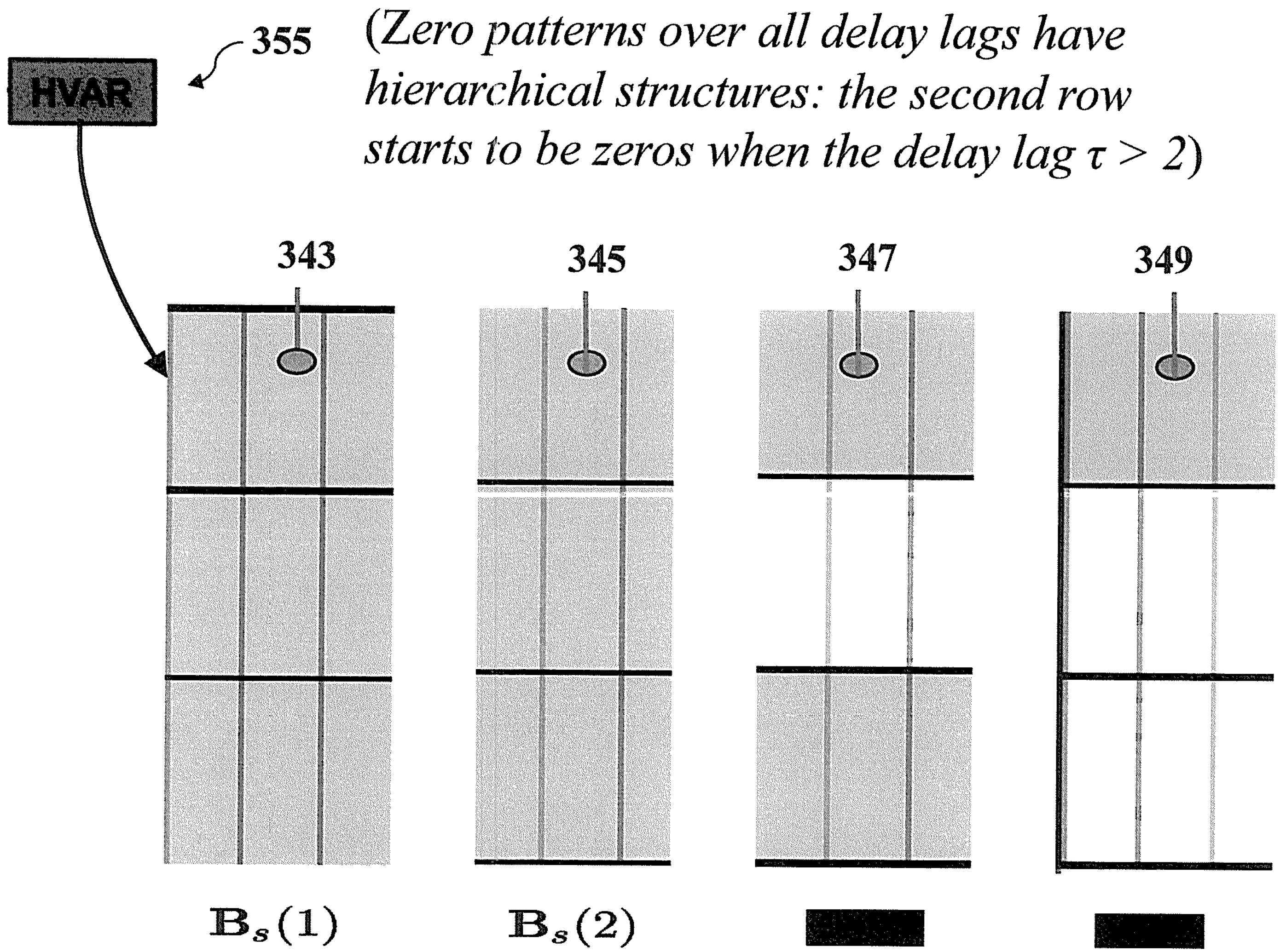
HVAR
355
(Zero patterns over all delay lags have hierarchical structures: the second row starts to be zeros when the delay lag $\tau > 2$)
343
345
347
349
$\mathbf{B}_s(1)$
$\mathbf{B}_s(2)$
[redacted]
[redacted]

FIG. 3D

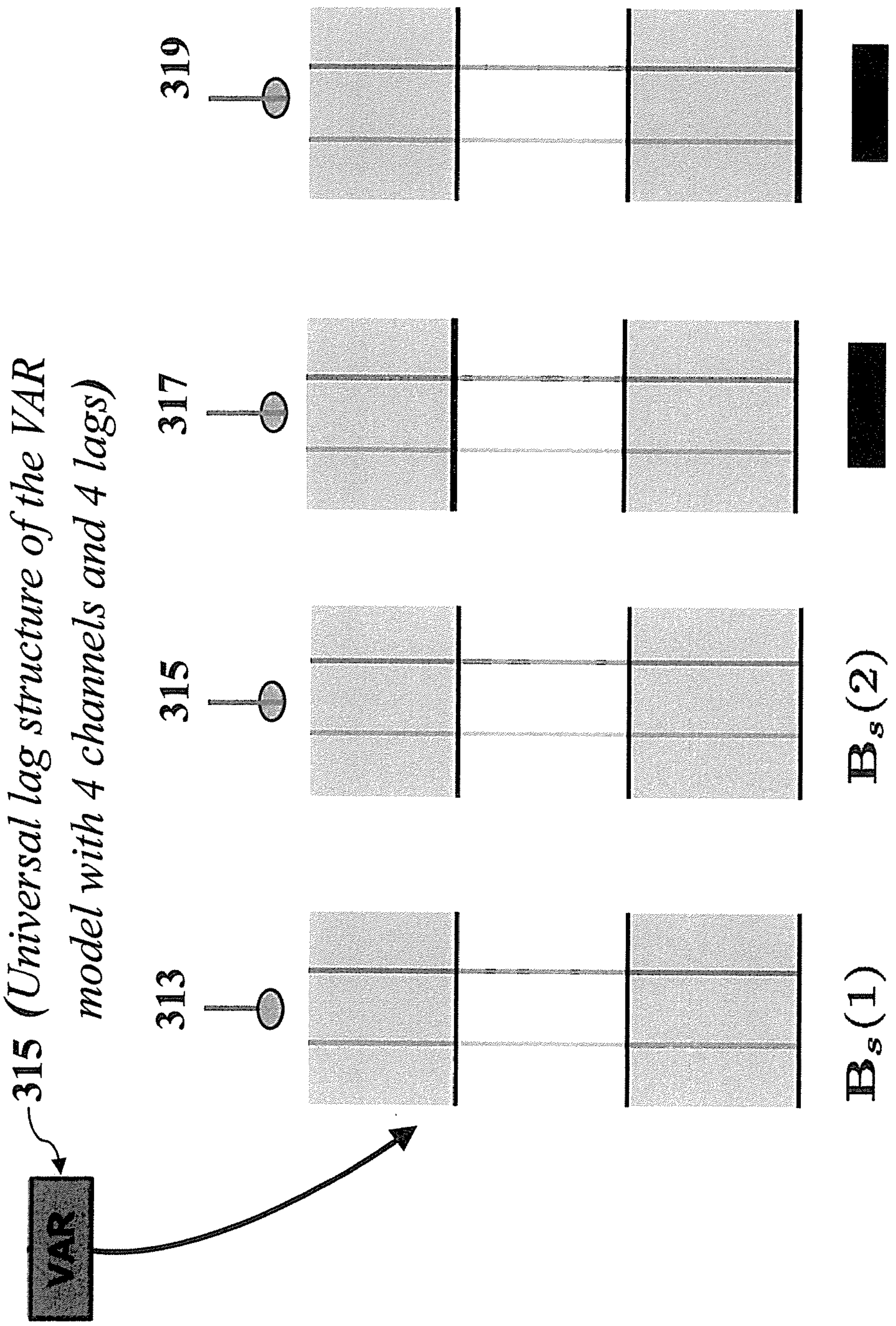
VAR
315 (Universal lag structure of the VAR model with 4 channels and 4 lags)
313
315
317
319
$B_s(1)$
$B_s(2)$

FIG. 3E

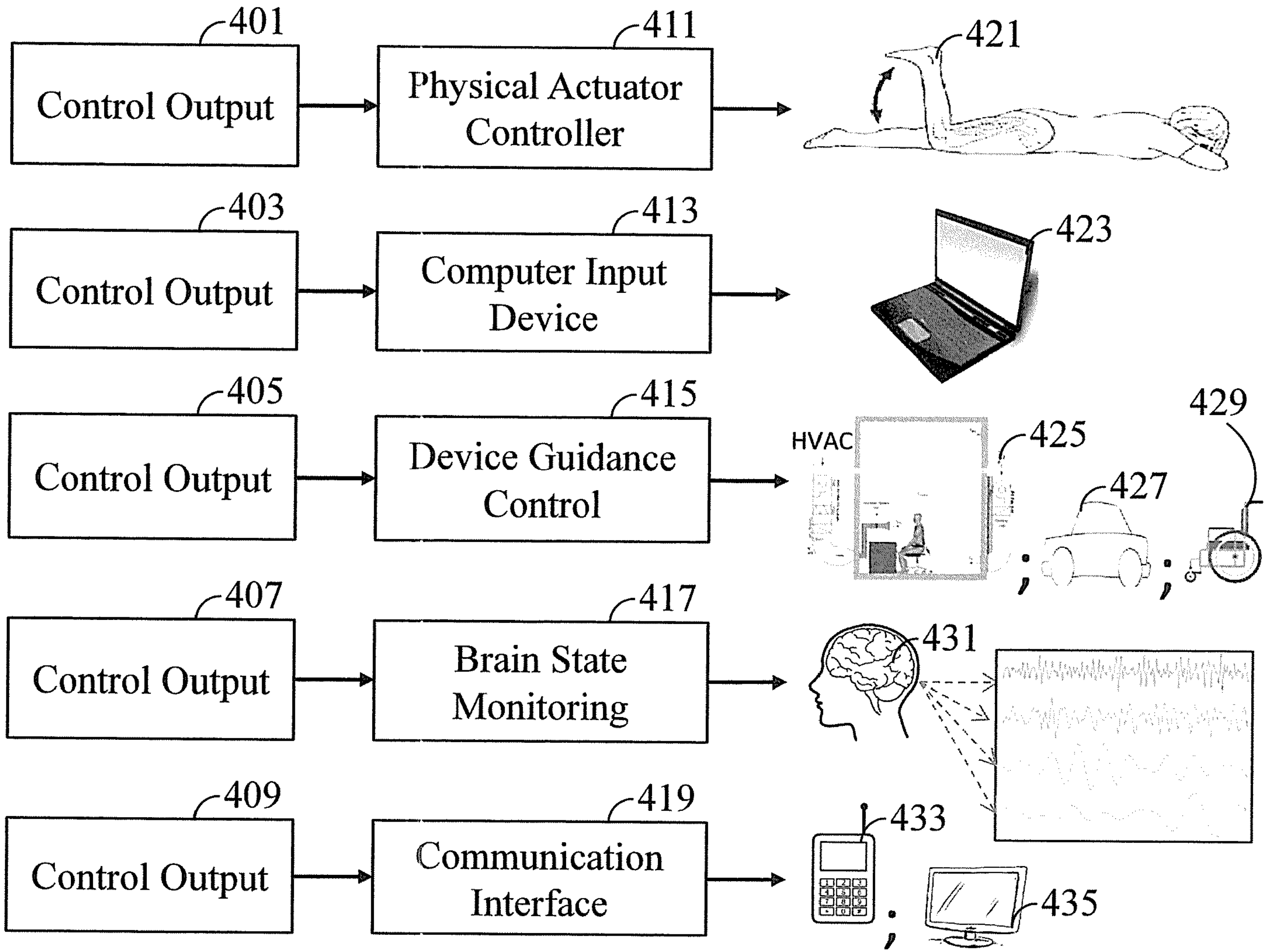

401 Control Output
403 Control Output
405 Control Output
407 Control Output
409 Control Output
411 Physical Actuator Controller
413 Computer Input Device
415 Device Guidance Control
417 Brain State Monitoring
419 Communication Interface
421
423
HVAC
425
427
429
431
433
435


FIG. 4

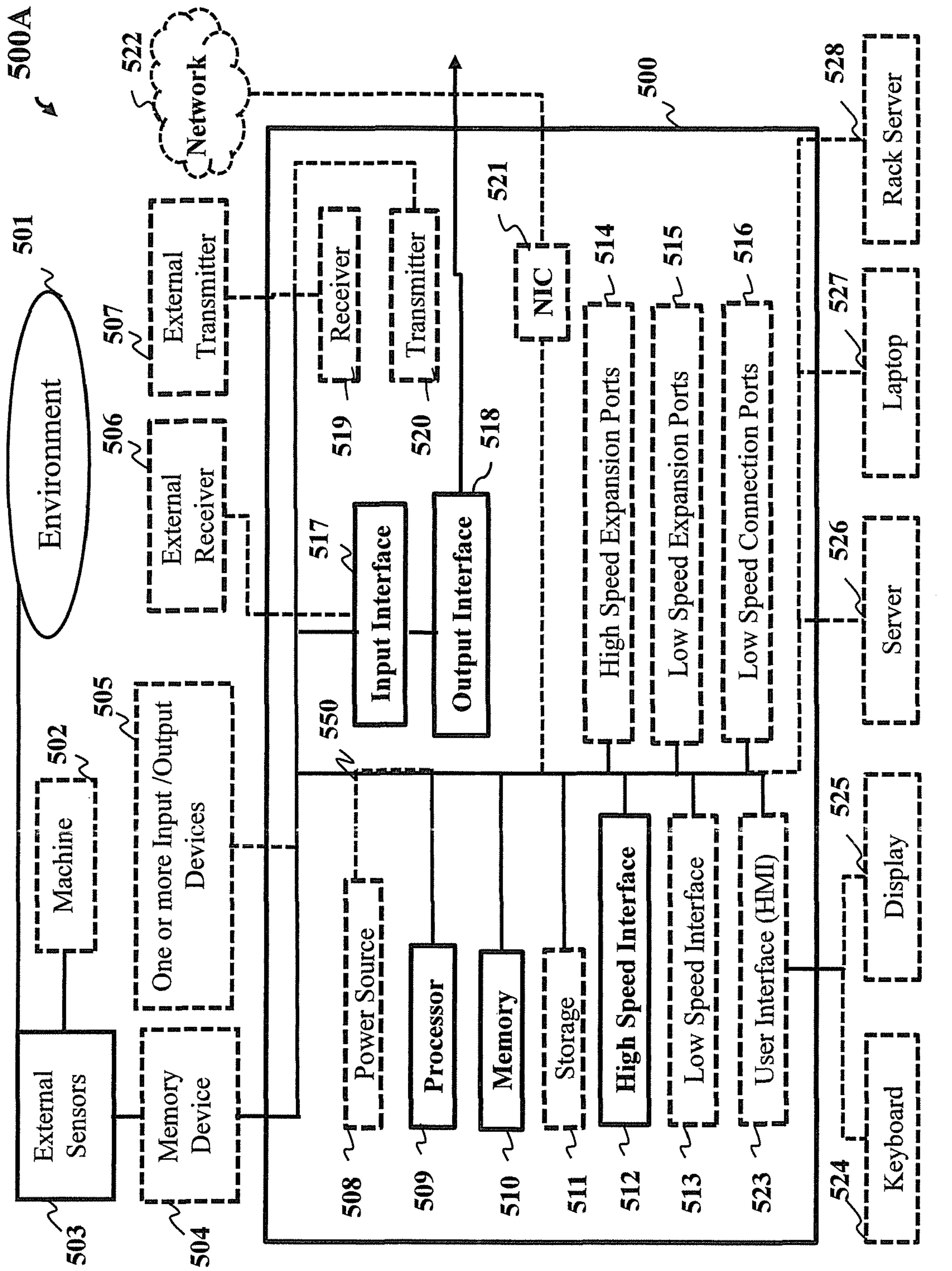
500A
Environment
501
Network
522
External Transmitter
507
External Receiver
506
Machine
502
External Sensors
503
One or more Input /Output Devices
505
Memory Device
504
500
Receiver
519
Transmitter
520
Input Interface
517
Output Interface
518
NIC
521
High Speed Expansion Ports
514
Low Speed Expansion Ports
515
Low Speed Connection Ports
516
550
Power Source
508
Processor
509
Memory
510
Storage
511
High Speed Interface
512
Low Speed Interface
513
User Interface (HMI)
523
Keyboard
524
Display
525
Server
526
Laptop
527
Rack Server
528

FIG. 5A

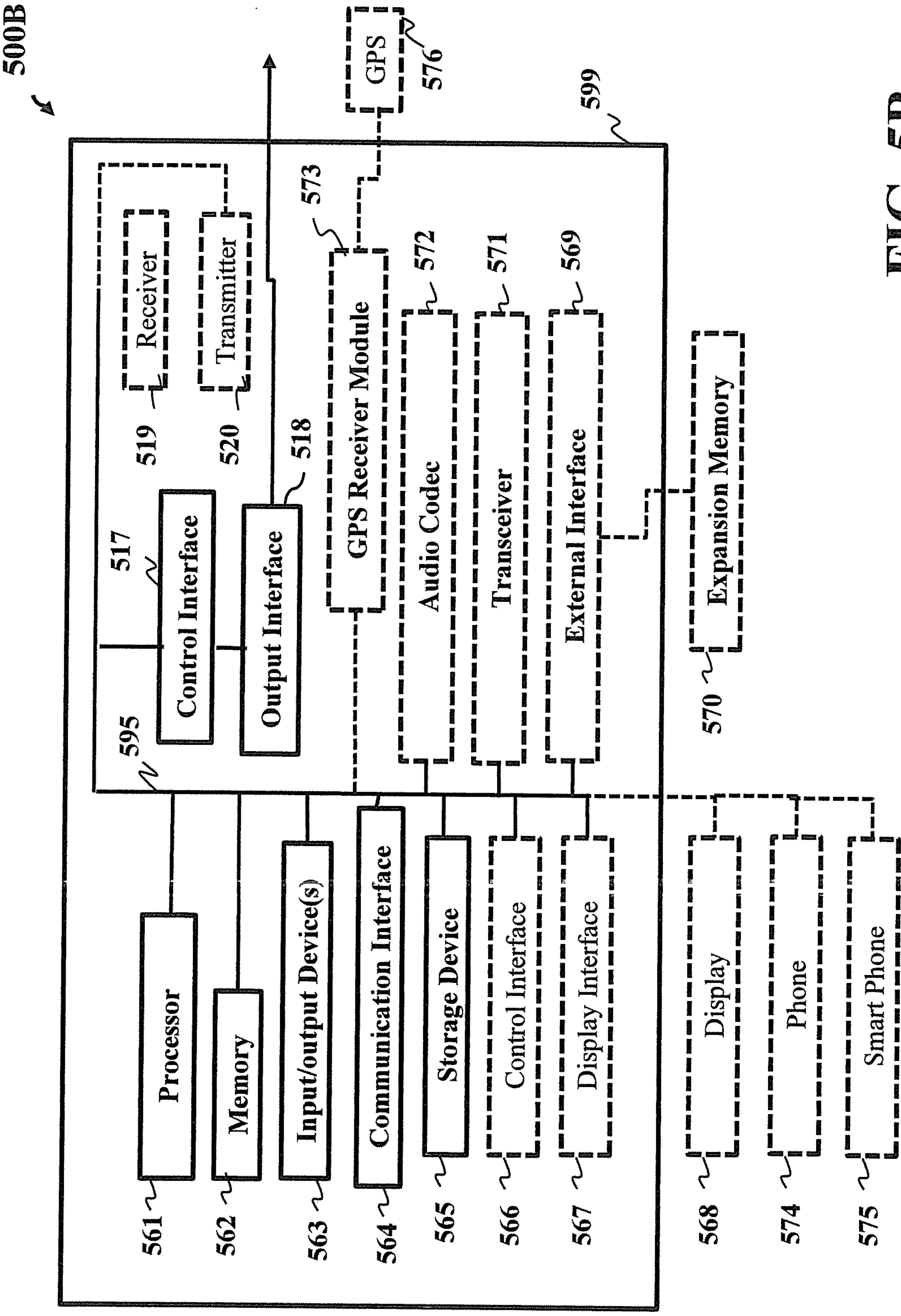
500B
Processor
561
Memory
562
Input/output Device(s)
563
Communication Interface
564
Storage Device
565
Control Interface
566
Display Interface
567
Display
568
Phone
574
Smart Phone
575
595
Control Interface
517
Output Interface
518
Receiver
519
Transmitter
520
GPS Receiver Module
573
GPS
576
Audio Codec
572
Transceiver
571
External Interface
569
Expansion Memory
570
599

FIG. 5B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140171757 A1
- WO 2012153965 A2
- US 20140236039 A1
- CN 108433722 A